(19) Europäisches Patentamt
European Patent Office
Office européen des brevets

(11) Veröffentlichungsnummer: **0 203 450 B1**

(12) # EUROPÄISCHE PATENTSCHRIFT

(45) Veröffentlichungstag der Patentschrift: **01.04.92**

(51) Int. Cl.$^5$: **C07K 5/06**, C07D 209/52, A61K 37/02

(21) Anmeldenummer: **86106515.9**

(22) Anmeldetag: **14.05.86**

Die Akte enthält technische Angaben, die nach dem Eingang der Anmeldung eingereicht wurden und die nicht in dieser Patentschrift enthalten sind.

(54) **Neue Derivate bicyclischer Aminosäuren, Verfahren zu ihrer Herstellung, diese enthaltende Mittel und deren Verwendung.**

(30) Priorität: **23.05.85 DE 3518514**

(43) Veröffentlichungstag der Anmeldung:
**03.12.86 Patentblatt 86/49**

(45) Bekanntmachung des Hinweises auf die Patenterteilung:
**01.04.92 Patentblatt 92/14**

(84) Benannte Vertragsstaaten:
**AT BE CH DE FR GB IT LI LU NL SE**

(56) Entgegenhaltungen:
**EP-A- 0 089 637**
**EP-A- 0 135 181**
**WO-A-86/00896**

(73) Patentinhaber: **HOECHST AKTIENGESELLSCHAFT**
**Postfach 80 03 20**
**W-6230 Frankfurt am Main 80(DE)**

(72) Erfinder: **Urbach, Hansjörg, Dr.**
**Le Lavandoustrasse 41**
**W-6242 Kronberg/Taunus(DE)**
Erfinder: **Henning, Rainer, Dr.**
**Rotenhofstrasse 31**
**W-6234 Hattersheim am Main(DE)**
Erfinder: **Becker, Reinhard, Dr.**
**Adelheidstrasse 101**
**W-6200 Wiesbaden(DE)**

EP 0 203 450 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus EP-A 089 637 und EP-A 135 181 sind Derivate bicyclischer Aminosäuren bekannt, die zur Bekämpfung des Bluthochdrucks geeignet sind. In EP-A 089 637 werden Hexahydroindol-Derivate beschrieben und Angaben zu deren Synthese und Verwendung gemacht.

Die vorliegende Erfindung betrifft die Bereitstellung neuer Derivate der Azabicyclooctencarbonsäure der Formel (I), welche bereits in deutlich geringerer Dosierung zu einer Blutdrucksenkung führen.

Gegenstand der Erfindung sind daher neue Derivate der bicyclischen Aminosäuren der Formel I

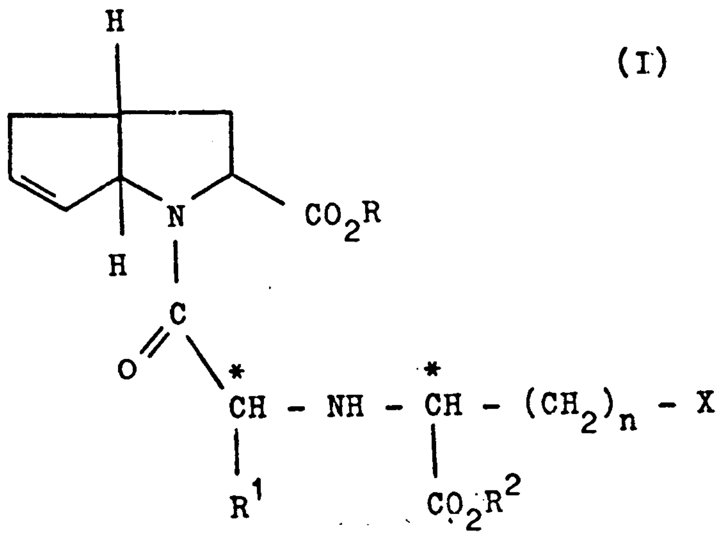

(I)

in der

n = 0, 1 oder 2,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl,($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, Phenyl, Naphtyl oder teilhydriertes Phenylode Naphtyl, das jeweils durch ($C_1$ bis $C_4$)-Alkyl, ($C_1$ oder $C_2$)-Alkoxy oder Halogen substituiert sein kann, Aryl-($C_1$ bis $C_4$)-alkyl oder Aroyl-$C_1$-alkyl, die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, ($C_1$ bis $C_6$)-Alkyl, ($C_2$ bis $C_6$)-Alkenyl oder Aryl-($C_1$ bis $C_4$)-alkyl,

X = ($C_1$ bis $C_6$)-Alkyl, ($C_2$ bis $C_6$)-Alkenyl, ($C_5$ bis $C_9$)-Cycloalkyl, Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkyl-amino oder Methylendioxy mono-, di- oder trisubstituiert sein kann, oder 3-Indolyl bedeuten,

sowie deren physiologisch unbedenkliche Salze.

Als Salze kommen insbesondere Alkali- oder Erdalkalisalze, Salze mit physiologisch verträglichen Aminen und Salze mit anorganischen oder organischen Säuren wie z. B. HCl, HBr, $H_2SO_4$, Maleinsäure, Fumarsäure in Frage.

Unter Aryl ist hier wie im folgenden gegebenenfalls substituiertes Phenyl oder Naphthyl zu verstehen. Alkyl kann geradkettig oder verzweigt sein.

In der bevorzugten Konfiguration der H-Atome an C-1 und C-5 des Bicyclus kommen zwei mögliche Konfigurationen der Carboxygruppe in Betracht, und zwar die exo-Stellung (Formelrest Ia) und die endo-Stellung (Formelrest Ib) der Carboxygruppe.

Die endo-Stellung der Carboxygruppe an C-3 ist so definiert, daß die Carboxygruppe in Richtung des ungesättigten Fünfringes des Bicyclus, d.h. der konkaven Seite des Bicyclus zugewandt ist (Formelrest Ib).

Entsprechend ist die exo-Stellung der Carboxygruppe an C-3 so definiert, daß die Carboxygruppe in Richtung der betreffenden Brückenkopf-H-Atome orientiert ist (Formelrest Ia).

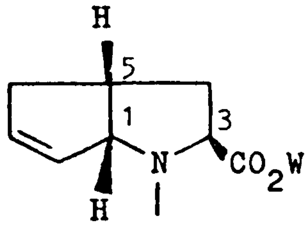

(Ia)

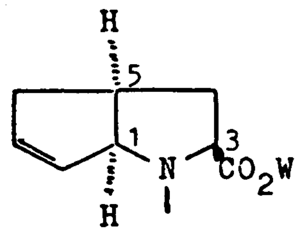

(Ib)

Verbindungen der Formel I besitzen chirale C-Atome in den Positionen C-1, C-3, C-5, sowie in den mit einem Stern markierten C-Atomen der Seitenkette. Sowohl die R- als auch die S-Konfigurationen an allen Zentren sind Gegenstand der Erfindung. Die Verbindungen der Formel I können daher als optische Isomere, als Diastomere, als Racemate oder als Gemische derselben vorliegen. Bevorzugt sind jedoch die Verbindungen der Formel I, in denen das C-Atom 3 im bicyclischen Ringsystem, sowie die mit einem Stern (*) markierten C-Atome der Seitenkette S-Konfiguration aufweisen.

Besonders bevorzugte Verbindungen der Formel I sind solche, in denen

n = 2

R = Wasserstoff oder Alkyl mit 1 bis 4 C-Atomen,

$R^1$ = Wasserstoff, ($C_1$ bis $C_3$)-Alkyl, ($C_2$ oder $C_3$)-Alkenyl, Benzyl, Phenethyl, 4-Amino-butyl oder Benzoylmethyl,

$R^2$ = Wasserstoff, ($C_1$ bis $C_4$)-Alkyl oder Benzyl und

X = Methyl, Cyclohexyl, Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkyl-amino, Nitro oder Methylendioxy, mono- oder disubstituiert oder im Falle von Methoxy trisubstituiert sein kann, bedeuten,

insbesondere solche Verbindungen der Formel I, in denen n = 2, R = Wasserstoff, $R^1$ = Methyl, X = Phenyl, Methyl oder Cyclohexyl, $R^2$ = Wasserstoff oder Ethyl bedeuten, der Bicyclus die cis-Konfiguration besitzt, die Carboxylgruppe exo- oder endo-orientiert ist und die chiralen C-Atome, die mit einem Stern (*) gekennzeichnet sind, und C-Atom 3 die S-Konfiguration besitzen.

Die Erfindung betrifft ferner ein Verfahren zur Herstellung der Verbindungen der Formel I. Eine Verfahrensvariante ist dadurch gekennzeichnet, daß man eine Verbindung der Formel II

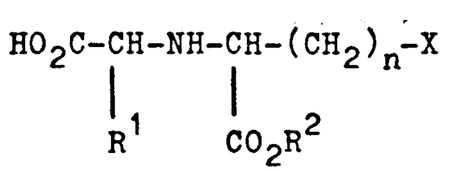

(II)

worin n, $R^1$, $R^2$ und X die Bedeutungen wie in Formel I haben, mit einer Verbindung der Formel III,

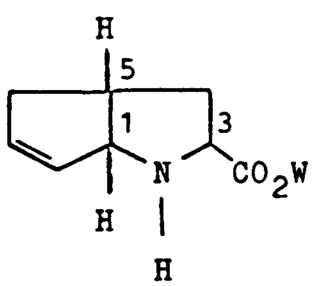

(III)

in welcher

W = Wasserstoff oder einen sauer oder basisch abspaltbaren Rest, insbesondere einen tert.-Butyl-Rest bedeuten,

nach bekannten Amidbildungsmethoden der Peptidchemie umsetzt und gegebenenfalls anschließend durch

Säurebehandlung den Rest W und gegebenenfalls durch zusätzliche Säure- oder Basenbehandlung auch den Rest $R^2$ abspaltet, wobei jeweils die freien Carbonsäuren erhalten werden.

Ferner können Verbindungen der Formel I auch in der Weise hergestellt werden, daß man eine Verbindung der Formel IV

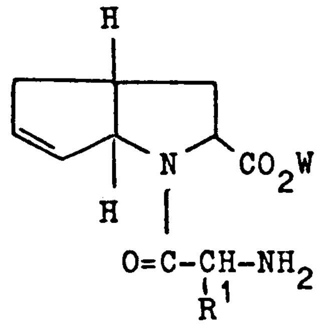

(IV),

in welcher $R^1$ die Bedeutung wie in Formel I und W die Bedeutung wie in Formel III besitzen, gemäß der in J. Amer. Chem. Soc. 93, 2897 (1971) beschriebenen Verfahrensweise mit einer Verbindung der Formel V

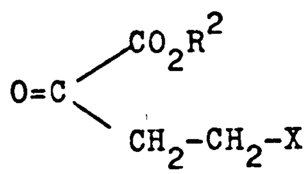

(V)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann elektrolytisch oder mit Reduktionsmitteln wie beispielsweise Natriumborhydrid oder Natriumcyanborhydrid erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$-$C_9$)-Aralkyl bedeutet, überführt werden.

Die Erfindung betrifft auch Verbindungen der Formel III,

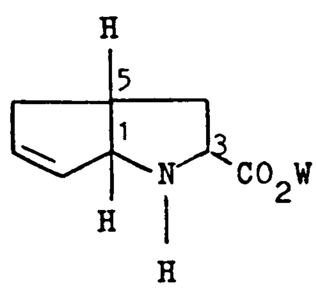

(III),

in der die H-Atome an den C-Atomen 1 und 5 zueinander cis-konfiguriert sind und die Gruppe $-CO_2W$ an C-Atom 3 exo- oder endo-ständig zum bicyclischen Ringsystem orientiert ist und worin

W = Wasserstoff oder einen sauer abspaltbaren Rest bedeutet.

Diese Verbindungen dienen gemäß der Erfindung als Ausgangsstoffe bei der Synthese von Verbindungen der Formel I und können erfindungsgemäß nach folgender Verfahrensweise hergestellt werden: Verbindungen der Formel (VI) und (VII),

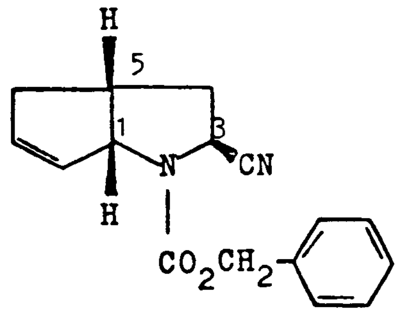

(VI)

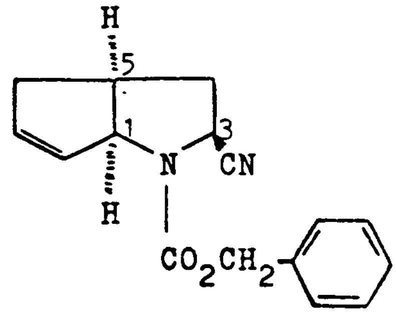

(VII)

in der die Wasserstoffatome an den C-Atomen 1 und 5 zueinander cis-konfiguriert sind und die Nitrilgruppe an C-Atom 3 der Verbindung der Formel (VI) exo-ständig und der Verbindung der Formel (VII) endoständig steht, sind in der Literatur beschrieben (D.A. Evans et al., Tetrahedron Letters Vol. 26, 1907 (1985). Diese Verbindungen werden unter sauren oder alkalischen Bedingungen zu Verbindungen der Formel III, in der W Wasserstoff bedeutet, verseift.

So wird z.B. die Verbindung der Formel VI zweckmäßigerweise mit konzentrierter Bromwasserstoffsäure unter Rückfluß zur Verbindung der Formel (IIIa),

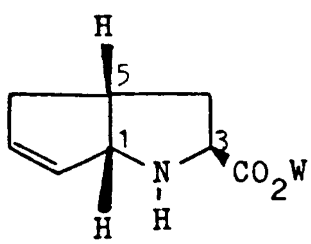

(IIIa)

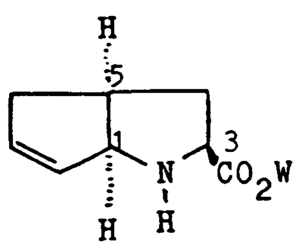

(IIIb)

in der W Wasserstoff bedeutet, die Wasserstoffatome an den C-Atomen 1 und 5 zueinander cis-konfiguriert sind und die $CO_2W$-Gruppe an C-Atom 3 exo-ständig zum olefinischen Fünfring steht, verseift. Entsprechend entsteht aus der Verbindung VII die Verbindung der Formel IIIb, in der W Wasserstoff bedeutet, die Wasserstoffatome an den C-Atomen 1 und 5 zueinander cis-konfiguriert sind und die $CO_2W$-Gruppe an C-Atom 3 endo-ständig zum olefinischen Fünfring steht.

Die Verseifung kann auch mit konzentrierter oder mit wasser- oder alkoholverdünnter Salzsäure durchgeführt werden. Auch verdünnte Schwefelsäure ist anwendbar. Die Verseifung unter basischen Bedingungen geschieht vorzugsweise mit wäßriger oder alkoholisch/wäßriger Natronlauge oder Kalilauge.

In analoger Weise lassen sich nach der Herstellvorschrift der Tetrahedron Letters Publication 26, 1907 (1985) weitere N-Acylderivate der Formel VIa und VIIa herstellen, in der $R^3$ für ($C_1$-$C_6$)-Alkyl, ($C_5$-$C_9$)-Cycloalkyl, ($C_2$-$C_6$)-Alkenyl, ($C_1$-$C_6$)-Alkoxy, Aryl, Aryloxy, Aryl-($C_1$-$C_4$)-alkyl oder Aryl-($C_1$-$C_4$)-alkoxy steht, die unter basischen oder sauren Bedingungen zu Verbindungen der Formel IIIa und IIIb, in der W Wasserstoff bedeutet, hydrolysiert werden können.

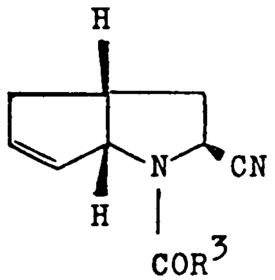

(VIa)

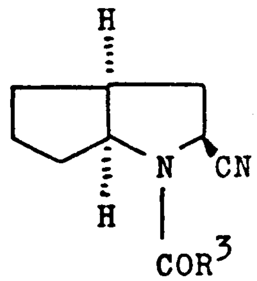

(VIIa)

So lassen sich z. B. die N-tert. Butoxycarbonylverbindungen der Formel VIa und VIIa ($R^3$ = $-O-C(CH_3)_3$) herstellen, die unter sauren Bedingungen (z.B. mit konz. Bromwasserstoffsäure) zu den Verbindungen der Formel IIIa und IIIb mit W = Wasserstoff verseift werden.

Die endo-cis-Verbindungen IIIb und die exo-cis-Verbindungen IIIa liegen jeweils als Racemate vor. Die Aminosäuren können gegebenenfalls verestert werden. Die bevorzugten tert.-Butylester der Aminosäuren der Formel III (W = tert.-Butyl) werden nach den in der Peptidchemie üblichen Methoden erhalten, wie z. B. durch Reaktion der Säuren mit Isobutylen in einem inerten organischen Lösungsmittel (z.B. Dioxan) in Gegenwart von Säuren (wie z.B. Schwefelsäure). Als besonders vorteilhaft hat sich das folgende Verfahren erwiesen:

Die entsprechende Aminosäure wird mit einer basisch abspaltbaren Gruppe, wie z. B. der Methylsulfonylethoxycarbonylgruppe (= MSC), (Tesser, Balvert-Geers, Int. J. Pept. Protein Res 7, 295 (1975)) oder der 9-Fluorenylmethyl-oxycarbonylgruppe (= FMOC) am Stickstoff acyliert.

Die Carbonsäure wird im neutralen bis schwach basischen pH-Bereich mit tert.-Butanol in einem organischen Lösungsmittel, wie z. B. Pyridin, in Gegenwart von n-Propylphosphonsäureanhydrid zum entsprechenden tert.-Butylester umgesetzt. Der tert.-Butylester kann auch durch Umsetzung z.B. des FMOC-Carbonsäurederivats mit tert.-Butanol in Gegenwart von Phosphoroxychlorid erhalten werden. Durch Abspaltung der MSC- oder FMOC-Schutzgruppe im stark alkalischen pH-Bereich mit Alkali im wäßrigen Lösungsmittel oder einer organischen Base im organischen Lösungsmittel wird der tert.-Butylester der Formel III erhalten (W = tert. Butyl).

Die als Ausgangsstoffe zur Herstellung der Verbindungen der Formel I verwendeten Verbindungen der Formel II mit n = 2, $R^1$ = Methyl und $R^2$ = Methyl oder Ethyl und X = Phenyl sind bekannt (europäische Patentanmeldung Nr. 37 231). Desgleichen sind Verbindungen der Formel II mit n = 2, $R^1$ und X = $CH_3$ und $R^2$ = $C_2H_5$ bekannt (Tetrahedr. Lett. 23, (1982), 1677). Die Verbindungen der Formel II lassen sich nach verschiedenen Verfahrensweisen herstellen. Eine Synthesevariante geht von einem Keton der unten genannten Formel VIII aus, das nach bekannten Verfahrensweisen in einer Mannich-Reaktion mit einer Verbindung der unten genannten Formel IX zusammen mit Aminosäureestern der Formel X

$X-CO-CH_3$

(VIII)

$OHC-CO_2R^2$

(IX)

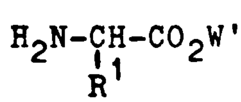

(X)

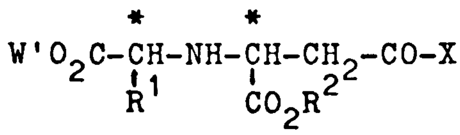

(XI)

worin $R^1$ die obengenannte Bedeutung besitzt und W' einen hydrogenolytisch oder sauer abspaltbaren Rest, insbesondere einen Benzyl- oder einen tert.-Butyl-Rest bedeutet, zu einer Verbindung der Formel XI , worin $R^1$, $R^2$, X und W' die obengenannten Bedeutungen besitzen, mit der Einschränkung, daß, wenn W' einen hydrogenolytisch abspaltbaren Rest, insbesondere Benzyl, bedeutet, $R^2$ nicht die Bedeutung von W' besitzen darf, umsetzt. Spaltet man den Rest W' hydrogenolytisch mit Hilfe von beispielsweise Palladium ab, werden bei einer Wasserstoffaufnahme von 3 Moläquivalenten Verbindungen der Formel II erhalten.

Verbindungen der Formel XI sind auch durch Michael-Additionen einer Verbindung der Formel XII

$R^2O_2C - CH = CH - COX$ (XII)

mit einer Verbindung der obengenannten Formel X nach bekannten Verfahrensweisen zugänglich. Bevorzugt eignet sich dieses Verfahren zur Herstellung von solchen Verbindungen der Formel XI, in denen $R^1$ = Methyl, $R^2$ = Ethyl und X = Aryl bedeuten.

Die Verbindungen der Formel XI fallen als Diastereomerengemische an. Bevorzugte Diastereomeren der Formel XI sind solche, in denen die mit einem Stern markierten chiralen C-Atome jeweils S-Konfiguration aufweisen. Diese können beispielsweise durch Kristallisation oder durch Chromatographie, z. B. an Kieselgel, abgetrennt werden. Bei der nachfolgenden Abspaltung des Restes W' bleiben die Konfiguration der chiralen C-Atome erhalten.

Die als Ausgangsstoffe zur Herstellung der Verbindungen der Formel I verwendeten Verbindungen der obengenannten Formel IV werden aus den Verbindungen der obengenannten Formel III durch Umsetzen mit einer N-geschützten 2-Aminocarbonsäure der Formel XIII

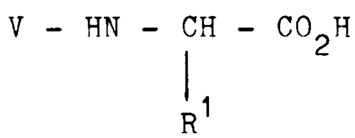

(XIII),

worin V eine Schutzgruppe bedeutet und $R^1$ die obengenannte Bedeutung besitzt, nach bekannten Verfahrensweisen erhalten. Als Schutzgruppe V, die nach beendeter Reaktion wieder abgespalten wird, kommt beispielsweise tert.-Butoxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol oder n-Propanphosphonsäureanhydrid oder Methyl-ethylphosphinsäureanhydrid zugesetzt werden. Bei der nachfolgenden sauren Abspaltung des Restes W werden als Säuren bevorzugt Trifluoressigsäure oder Chlorwasserstoff eingesetzt.

In der oben beschriebenen Reaktion zur Herstellung der Verbindungen der Formeln III, IV und I bleiben jeweils die Konfigurationen der Zwischenprodukte an den Brückenkopf-C-Atomen 1 und 5 erhalten.

Die Verbindungen der Formel III fallen als racemische Gemische an und können als solche in die weiteren, oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantiomere eingesetzt werden. Die reinen Enantiomeren können auch erhalten werden. Fallen die Verbindungen der Formel I als Racemate an, können auch diese nach den üblichen Methoden wie beispielsweise über Salzbildung mit optisch aktiven Basen oder Säuren in ihre Enantiomeren gespalten oder durch Chromatographie getrennt werden.

Die erfindungsgemäßen Verbindungen der Formel I liegen, falls R = Wasserstoff ist, als innere Salze vor. Als amphotere Verbindungen können sie Salze mit Säuren oder Basen bilden. Diese Salze werden in

üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z. B. in Erhardt-Ruschig, Arzneimittel, 2. Auflage, Weinheim, 1972, beschrieben. Die Verbindungen der Formel I können auch zur Bekämpfung der Koronaren Herzinsuffizienz verschiedener Genese eingesetzt werden. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen Patienten. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel für die neuen aktiven Verbindungen und die entsprechenden physiologisch verträglichen Salze kommen z. B. in Frage: Wasser, physiologische Kochsalzlösungen oder Alkohole, z. B. Ethanol, Propandiol oder Glycerin, daneben auch Zuckerlösungen wie Glucose- oder Mannitlösungen, oder auch eine Mischung aus den verschiedenen genannten Lösungsmitteln.

Die in den folgenden Beispielen angegebenen $^{1}$H-NMR-Daten wurden, wenn nicht anders angegeben, in $CDCl_3$ ermittelt und sind in $\delta$ (ppm) angegeben.

Beispiel 1

2-[N-[(S)-1-Ethoxycarbonyl-3-phenylpropyl]-L-alanyl]-(1S,3S,5S)-2-azabicyclo[3.3.0]-7-octen-3-carbonsäure

a) 1SR,3SR,5SR-2-Azabicyclo-[3.3.0]-7-octen-3-carbonsäure (cis-endo-2-azabicyclo[3.3.0]-7-octen-3-carbonsäure)

1 g N-Benzyloxycarbonyl-1SR,3SR,5SR-2-azabicyclo[3.3.0]-7-octen-3-carbonsäurenitril werden mit 10 ml konz. Bromwasserstoffsäure auf 60-70 °C erhitzt. Nach Ende der Verseifung wird im Vakuum eingedampft und anschließend zweimal mit Toluol am $^{(R)}$ Rotavapor eingedampft. Der Rückstand wird in Wasser aufgenommen und mit Ionenaustauscher (z.B. IRA 93) auf pH 4 gebracht. Nach Abtrennen des Ionenaustauschers wird die wäßrige Lösung eingedampft und der Rückstand über Kieselgel mit Methylenchlorid/Methanol/Eisessig/Wasser (20:10:0.5:0.5) bzw. Ethylacetat/Methanol 1:1, dann Methanol gereinigt.

Ausbeute: 0.4 g, Fp. 254-256 °C (Zers.), $R_f$: 0,05 ($SiO_2$; Essigester/Methanol 1 : 1) $^{1}$H-NMR (270 MHz; $D_2O$;ppm): 1,8-2,0 (m,1H); 2,2-2,4 (m,1H); 2,1-2,3 (m,2H); 3,1-3,25 (m,1H); 4,1-4,2 (dd,1H); 4,8-4,9 (m,1H); 5,8 (m,1H); 6,1 (m, 1H).

b) N-Fluorenylmethyloxycarbonyl-1SR,3SR,5SR-2-azabicyclo-[3.3.0]-7-octen-3-carbonsäure

1,5 g Säure aus Beispiel 1 a werden in 22 ml Wasser/Dioxan (1:1) gelöst, 1,5 g $NaHCO_3$ und 3,7 g 9-Fluorenylmethyl-succinimidyl-carbonat (FMOC-ONSuc) zugegeben. Es wird 2 Tage bei Raumtemperatur gerührt. Die Temperatur kann zur Reaktionsbeschleunigung auf 35 °C erhöht werden. Nach der Reaktion wird das Dioxan im Vakuum entfernt, die wäßrige Lösung auf pH 3,5 angesäuert, mit Ethylacetat extrahiert, die Ethylacetatlösung getrocknet und im Vakuum eingeengt.

Ausbeute: 3,2 g, $R_f$:0,64 ($SiO_2$; Ethylacetat/Methanol 1:1; $J_2$), $^{1}$H-NMR (270 MHz, $CD_3OD$; ppm): 1,6-3,0 (m,

5H); 4,0-4,9 (m, 5H); 5,45-6,0 (m, 2H); 7,25-7,9 (m, 8H).

$c_1$) N-Fluorenylmethyloxycarbonyl-1SR,3SR,5SR-2-azabicyclo-[3.3.0]-7-octen-3-carbonsäure-tert.-butylester

3,0 g Säure aus Beispiel 1 b werden bei -10 °C mit 8 ml Pyridin und 20 ml tert.-Butanol versetzt und dazu 0,85 ml Phosphoroxychlorid gegeben. Nach 30 Min. wird die Kühlung entfernt und bei Raumtemperatur, dann bei 30 °C gerührt. Nach der Reaktion wird auf wäßrige Natriumhydrogencarbonatlösung gegossen, mit Ethylacetat extrahiert, der Extrakt getrocknet und anschließend im Vakuum eingedampft. Der Rückstand wird über $SiO_2$ mit Cyclohexan/Ethylacetat 4:1 chromatographiert.
Ausbeute: 1,2 g.
$R_f$: 0,6 ($SiO_2$; Cyclohexan/Ethylacetat 1:1; $J_2$-Anfärbung)

$c_2$) Es werden 10 ml Isobutylen kondensiert, 270 mg der Verbindung aus Beispiel 1b) in 2 ml Methylenchlorid gelöst dazugegeben und mit 0,1 ml konz. Schwefelsäure versetzt. Man läßt 48 Stunden bei 10 bar Stickstoffdruck und Raumtemperatur im Autoklaven. Nach der Reaktion wird in Methylenchlorid aufgenommen, mit 5 %iger wäßriger $Na_2CO_3$-Lösung versetzt und die Methylenchloridphase im Vakuum eingeengt. Der Rückstand wird mit wenig Wasser verdünnt, die wäßrige Lösung 3 mal mit Ethylacetat extrahiert, die organische Phase über $MgSO_4$ getrocknet und nach Filtrieren im Vakuum eingedampft. Der Rückstand wird über Kieselgel mit Cyclohexan/Ethylacetat 85 : 15 gereinigt.
Ausbeute: 170 mg; $R_f$: 0,78 ($SiO_2$; Methylenchlorid/Methanol 4 : 1, $J_2$)
$^1$H-NMR ($CDCl_3$; ppm): 1,35 (m,9H); 1,8 (m,1H); 2,1 (m,1H); 2,45 (m,2H); 2,82 (m,1H); 4,18 (m,2H); 4,37 (m,2H); 4,7 + 4,9 (je d,1H); 5,65 + 5,95 (je d,2H); 7,2-7,7 (m,8H).

d) 1SR,3SR,5SR-2-Azabicyclo[3.3.0]-7-octen-3-carbonsäure-tert.-butylester

3 g tert. Butylester aus Beispiel 1 c werden in 60 ml ≈ 2,5 n Diethylamin in Dimethylformamid bei Raumtemperatur 1 Stunde gerührt. Nach Beendigung der Reaktion (DC-Kontrolle) wird im Hochvakuum eingedampft und mit Diisopropylether verrieben. Der Rückstand wird über $SiO_2$ mit Essigester/Cyclohexan 1:1 chromatographiert.
Ausbeute: 0,7 g tert.-Butylester (m/e: 209)
$^1$H-NMR (270 MHz, $CDCl_3$; ppm): 1,4-1,6 (m,11H); 2,25-2,9 (m,3H); 3,6 (dd, 1H); 4,25-4,35 (m,1H); 5,6-5,75 (m,2H).

e) 2-[N-[(S)-1-Ethoxycarbonyl-3-phenylpropyl]-L-alanyl]-(1SR,3SR,5SR)-2-azabicyclo[3.3.0]-7-octen-3-carbonsäure-tert.-butylester

0,28 g N-(1-S-Carbethoxy-3-phenyl-propyl)-S-alanin werden in 4 ml Dimethylformamid gelöst. Bei Raumtemperatur werden 0,15 g Hydroxybenzotriazol und 0,22 g Dicyclohexylcarbodiimid zugegeben. Es wird 4 Stunden bei Raumtemperatur gerührt. Anschließend werden 0,24 g tert.-Butylester aus Beispiel 1 d zugegeben und 20 Stunden bei Raumtemperatur gerührt. Man verdünnt mit Essigester, saugt vom Harnstoff ab und dampft im Vakuum ein. Der Rückstand wird in Essigester aufgenommen, die Essigesterlösung mit Bicarbonatlösung gewaschen, getrocknet und eingeengt. Ausbeute: 0,4 g Öl (m/e: 470)

f) 2-[N-[(S)-1-Ethoxycarbonyl-3-phenylpropyl]-L-alanyl]-(1S,3S,5S)-2-azabicyclo[3.3.0]-7-octen-3-carbonsäure-tert.-butylester

Der ölige Rückstand aus Beispiel 1 e (0,4 g) wird über Kieselgel mit Essigester/Cyclohexan 2:1 bzw. Petrolether/Aceton 2:1 als Elutionsmittel in die Diastereomeren getrennt.

Man erhält 0,15 g des tert.-Butylesters mit der 3-S-endo-Konfiguration. $R_f$: 0,43; m/e 470; $[\alpha]_D^{20}$ : + 23,4 ° (c = 3, $CHCl_3$)

| | |
|---|---|
| $^1$H-NMR-Daten (270 MHz, $CDCl_3$; ppm): | 1,4 (s, 9H) |
| | 0,8 - 3,8 (m, 18H) |
| | 4,0 - 4,6 (m, 2H) |
| | 4,9 - 5,2 (m, 1H) |
| | 5,4 - 6,0 (m, 2H) |
| | 7,1-7,3 (m, 5H). |

Das entsprechende R,R,R,S,S-Isomer zeigt $[\alpha]_D^{20}$ : -51,4 ° (c = 5, $CHCl_3$).

g) 2-[N-[(S)-1-Ethoxycarbonyl-3-phenylpropyl)-L-alanyl]-(1SR,3SR,5SR)-2-azabicyclo[3.3.0]-7-octen-3-carbonsäure

0,15 g des tert.-Butylesters aus Beispiel 1 e werden in 1 ml Trifluoressigsäure bei 0 ° C gelöst und bei dieser Temperatur 3 Stunden gerührt. Die Trifluoressigsäure wird im Vakuum abgedampft und der Rückstand aus Diisopropylether kristallisiert.
Ausbeute an Trifluoracetat: 0,08 g.
Das Trifluoracetat wird mit basischem Ionenaustauscher ($OH^-$-Form) in Methanol/Wasser 60:40 in die Aminosäure überführt.
Ausbeute: 0,06 g (m/e: 486; nach Silylierung).

h) 2-[N-[(S)-1-Ethoxycarbonyl-3-phenylpropyl]-L-alanyl]-(1S,3S,5S)-2-azabicyclo[3.3.0]-7-octen-3-carbonsäure

Eine Lösung von 0,5 g tert.-Butylester aus Beispiel 1 f in 5 ml Methylenchlorid wird mit trockenem Chlorwasserstoffgas gesättigt und 16 Stunden bei 20 - 25 ° C stehengelassen. Die Lösung wird im Vakuum eingeengt. Der Rückstand wird mit Diisopropylether verrieben und abgesaugt.
Ausbeute: 0,4 g
Das Hydrochlorid wird mit basischem Ionenaustauscher ((R) Amberlite 7 RA 93) bei pH 4,0-4,5 in das Betain überführt.
Ausbeute: 0,3 g (m/e: 486 nach Silylierung 415 (FAB))
$^1$H-NMR (270 MHz, $CDCl_3$; ppm): 0,8-1,5 (m,6H); 1,7-3,2 (m,9H); 3,6-5,2 (m,6H); 5,6-6,1 (m,2H); 7,1-7,3 (m,5H). Das entsprechende R,R,R,S,S-Isomer zeigt $[\alpha]_D^{20}$ : -38,3 ° (c = 3,5; $CHCl_3$).

Beispiel 2

2-[N-[(S)-1-Ethoxycarbonyl-3-phenylpropyl]-L-alanyl]-(1R-3S,5R)-2-azabicyclo[3.3.0]-7-octen-3-carbonsäure

a) 1RS,3SR,5RS-2-Azabicyclo[3.3.0]-7-octen-3-carbonsäure-(cis-exo-2-Azabicyclo[3.3.0]-7-octen-3-carbonsäure)

1 g N-Benzyloxycarbonyl-1RS,3RS,5RS-2-azabicyclo[3.3.0]-7-octen-3-carbonsäurenitril werden analog Beispiel 1 a verseift.
Ausbeute: 0,5 g
$^1$H-NMR (270MHz, $D_2O$; ppm):2,1 (m,1H); 2,4 (m,2H); 2,8 (m,1H); 3,18 (m,1H); 4,05 (dd,1H); 4,92 (breites d,1H); 5,75 (m,1H); 6,23 (m,1H).

b) N-Fluorenylmethyloxycarbonyl-1RS,3SR,5RS-2-azabicyclo-[3.3.0]-7-octen-3-carbonsäure

1,5 g Säure aus Beispiel 2 a werden analog Beispiel 1 b umgesetzt.
Ausbeute: 3,0 g; $R_f$ = 0,54 ($SiO_2$; Ethylacetat/Methanol 1:1; $J_2$)

c) N-Fluorenylmethyloxycarbonyl-1RS,3SR,5RS-2-azabicyclo-[3.3.0]-7-octen-3-carbonsäure-tert.-butylester

3,0 g Säure aus Beispiel 2 b werden analog Beispiel 1 $c_2$) umgesetzt.
Ausbeute: 1,8 g (m/e: 431)
$^1$H-NMR (270 MHz, $CDCl_3$; ppm); 1,45 (d,9H); 1,87 (m,1H); 2,2 (m,2H); 2,55 (m,1H); 2,9 (m,1H); 4,15-4,6 (m,4H); 4,82 + 5,0 (je d,1H); 5,65 + 6,0 (je m,2H); 7,25-7,8 (m,8H).

d) 1RS,3SR,5RS-2-azabicyclo[3.3.0]-7-octen-3-carbonsäure-tert.-butylester

3 g tert.-Butylester aus Beispiel 2c werden analog dem in Beispiel 1 d beschreibenen Verfahren umgesetzt.
Ausbeute: 1,1 g (m/e: 209)
$^1$H-NMR (270 MHz,$CDCl_3$; ppm): 1,45 (s,9H); 1,8-2,0 (m,2H); 2,1-2,2 (m,1H); 2,88 (s,1H); 2,53-2,68 (m,1H); 2,7-2,9 (m,1H); 3,5-3,58 (dd,1H); 4,52 (m,1H); 5,57 (m,1H); 5,72 (m,1H).

e) 2-[N-[(S)-1-Ethoxycarbonyl-3-phenylpropyl]-L-alanyl]-(1R,3S,5R)-2-azabicyclo[3.3.0]-7-octen-3-carbonsäure-tert.-butylester

0,3 g tert.-Butylester aus Beispiel 2 d werden analog den Beispielen 1 e und 1 f umgesetzt.
Ausbeute: 0,25 g; $[\alpha]^{20}$ : -98° (c = 1, $CH_3OH$)

| | |
|---|---|
| $^1$H-NMR (270 MHz,$CDCl_3$; ppm): | 1,45 (s, 9H); |
| | 1,2 - 3,7 (m, 18H); |
| | 4,0 - 5,1 (m, 4H); |
| | 5,65 - 6,0 (m, 2H); |
| | 7,1 - 7,3 (s, 5H) |

Die isomere Verbindung 2-[N-[(S)-1-Ethoxycarbonyl-3-phenylpropyl]-L-alanyl]-(1S,3R,5S)-2-azabicyclo-[3.3.0]-7-octen-3-carbonsäure-tert.-butylester zeigt $[\alpha]_D^{20}$ : +86,4° (c = 1, $CH_3OH$).

f) 2-[N-[(S)-1-Ethoxycarbonyl-3-phenylpropyl]-L-alanyl]-(1R,3S,5R)-2-azabicyclo[3.3.0]-7-octen-3-carbonsäure

Diese Verbindung wird analog dem in Beispiel 1 h beschriebenen Verfahren aus 0,4 g tert.-Butylester des Beispiels 2 e hergestellt.
Ausbeute: 0,25 g (m/e: 486 nach Silylierung); $[\alpha]_D^{20}$ : - 161,8° (c = 1,5; $CH_3OH$).
$^1$H-NMR (270 MHz, DMSO-$d_6$; ppm): 1,0-1,3 (m,6H); 1,6-3,73 (m,9H); 4,0-4,15 (m,3H); 4,3 (d,1H); 4,8 (breites t,1H); 5,7 (m,1H); 5,84 (m,1H); 7,1-7,3 (m,5H).
Die isomere Verbindung 2-[N-[(S)-1-Ethoxycarbonyl-3-phenylpropyl]-L-alanyl]-(1S,3R,5S)-2-azabicyclo-[3.3.0]-7-octen-3-carbonsäure zeigt $[\alpha]_D^{20}$ : +110,3° (c = 2,5; $CH_3OH$).

Beispiel 3

2-[N-[(S)-1-Carboxy-3-phenylpropyl]-L-alanyl]-(1SR,3SR, 5SR)-2-azabicyclo-[3.3.0]-7-octen-3-carbonsäure

Eine Lösung von 0,1 g des im Beispiel 1 g hergestellten Ethylesters in 2 ml Wasser wird mit einem Äquivalent Kaliumhydroxid und einem 10%igen Überschuß 4n Kaliumhydroxid-Lösung versetzt. Nach 4-stündigem Rühren bei 20 bis 25°C wird die Reaktionslösung mit 2n Salzsäure auf einen pH-Wert von 4 eingestellt und im Vakuum eingeengt. Man nimmt den Rückstand in Essigester auf und filtriert das abgeschiedene Salz ab. Die Essigesterlösung wird eingeengt, der Rückstand mit Diisopropylether verrieben und abgesaugt.
Ausbeute: 0,08 g

| | |
|---|---|
| $^1$H-NMR-Daten: (nach H/D Austausch) | 1,1 (d, 3H) |
| | 1,0 - 3,8 (m, 9H) |
| | 3,9 - 4,8 (m, 4H) |
| | 5,6 - 6,0 (m, 2H) |
| | 7,1 - 7,3 (m, 5H) |

Beispiel 4

2-[N-[(S)-1-Carboxy-3-phenylpropyl]-L-alanyl]-(1S,3S,5S)-2-azabicyclo[3.3.0]-7-octen-3-carbonsäure

0,1 g Ethylester des Beispiels 1 h werden analog Beispiel 3 umgesetzt
Ausbeute: 0,08 g (m/e 386 = MG-$H_2O$)
$^1$H-NMR (DMSO-$d_6$; ppm); 1,2 (d,3H); 1,7-3,3 (m,9H); 3,6-5,2 (m,4H); 5,6-6,0 (m,2H); 7,1-7,3 (m,5H).

Beispiel 5

2-[N-[(S)-1-Carboxy-3-phenylpropyl]-L-alanyl]-(1R,3S,5R)-2-azabicyclo[3.3.0]-7-octen-3-carbonsäure

0,1 g Ethylester des Beispiels 2 f werden analog Beispiel 3 umgesetzt.
Ausbeute: 0,07 g (m/e: 368 = MG-$H_2O$)
$^1$H-NMR (DMSO-$d_6$; ppm): 1,1 (d,3H); 1,5-3,5 (m,9H); 3,7-5,2 (m,4H); 5,6-6,1 (m,2H); 7,1-7,3 (m,5H).

Beispiel 6

2-[$N_\alpha$-[(S)-1-Ethoxycarbonyl-3-phenylpropyl]-L-lysyl]-(1S,3S,5S)-2-azabicyclo[3.3.0]-7-octen-3-carbonsäure-dihydrochlorid

a) $N_{\alpha}$-[(S)-1-Ethoxycarbonyl-3-phenylpropyl]-$N_{\epsilon}$-tert. butoxycarbonyl-L-lysin

0,42 g (0,0002 Mol) D-2-Hydroxy-4-phenyl-buttersäureethylester und 0,16 ml trockenes Pyridin werden in 10 ml trockenem Methylenchlorid gelöst, auf 0 °C abgekühlt und 0,62 g Trifluormethansulfonsäureanhydrid in 3 ml abs. Methylenchlorid zugegeben. Es wird dann 2 Std. bei Raumtemperatur gerührt. Die Lösung wird mit Wasser gewaschen, getrocknet und im Vakuum eingeengt. Der Rückstand wird in 5 ml trockenem Methylenchlorid gelöst und zu einer Lösung von $N_{\epsilon}$-tert.butoxycarbonyl-L-lysinbenzylester und 0,27 ml Triethylamin in 10 ml trockenem Methylenchlorid zugetropft. Es wird 2 Std. bei Raumtemperatur gerührt. Danach wird mit Wasser gewaschen und die Methylenchloridlösung über $MgSO_4$ getrocknet und anschließend nach Entfernung des $MgSO_4$ im Vakuum eingeengt. Der Rückstand wird in Ethanol aufgenommen und mit Pd/C bei Normaldruck hydriert. Nach dem Absaugen des Katalysators wird die Lösung im Vakuum eingeengt.
Ausbeute: 0,6 g

| | |
|---|---|
| $^1$H-NMR ($D_2O$): | 1,4 (s, 9H); |
| | 1,0 - 1,4 (tr, 3H); |
| | 1,0 - 2,5 (m, 9H); |
| | 2,5 - 4,4 (m, 9H); |
| | 3,9 - 4,4 (g, 2H); |
| | 4,6 - 5,0 (m, 1H); |
| | 7,1 - 7,3 (m, 5H). |

b) 2-[$N_{\alpha}$-[(S)-1-Ethoxycarbonyl-3-phenylpropyl]-$N_{\epsilon}$-tert. butoxycarbonyl-L-lysyl]-(1S,3S,5S)-2-azabicyclo-[3.3.0]-7-octen-3-carbonsäure-tert.-butylester

0,6 g der Säure aus Beispiel 6 a, 1 Äquivalent des tert.-Butylesters aus Beispiel 1 d und 4 Äquivalente Triethylamin werden in 10 ml Methylenchlorid gelöst. Unter Eiskühlung werden 0,9 ml 50%ige n-Propanphosphonsäureanhydridlösung in Methylenchlorid zugegeben und bei Raumtemperatur über Nacht stehengelassen. Es wird nacheinander mit Wasser, wäßriger $KHSO_4$-Lösung, gesättigter $NaHCO_3$-Lösung und mit Wasser gewaschen. Danach wird getrocknet und im Vakuum eingedampft.
Ausbeute: 0,9 g Öl zweier diastereomerer Verbindungen. Das Diastereomerengemisch wird säulenchromatographisch mit Kieselgel und Cyclohexan/Essigester 2:1 getrennt. Das zuerst eluierte Isomere stellt obige Verbindung dar. Es werden 0,4 g Öl erhalten. (m/e: 627).

c) 2-[$N_{\alpha}$-[(S)-1-Ethoxycarbonyl-3-phenylpropyl]-L-lysyl]-(1S,3S,5S)-2-azabicyclo[3.3.0]-7-octen-3-carbonsäuredihydrochlorid

0,3 g der in Beispiel 6 b erhaltenen Verbindung werden analog Beispiel 1 h umgesetzt.
Ausbeute: 0,15 g

| | |
|---|---|
| $^1$H-NMR (nach H/D-Austausch): | 0,9 - 2,5 (m, 18H); |
| | 2,6 - 4,6 (m, 8H); |
| | 4,6 - 5,1 (m, 2H); |
| | 7,2 (s, 5H). |

Beispiel 7

2-[$N_{\alpha}$-[(S)-1-Carboxy-3-phenylpropyl]-L-lysyl]-(1S,3S,5S)-2-azabicyclo[3.3.0]-7-octen-3-carbonsäure

0,1 g der in Beispiel 6 c erhaltenen Verbindung werden analog Beispiel 3 umgesetzt.
Ausbeute: 0,08 g (m/e: 425; MG-$H_2O$).

Beispiel 8

2-[N-[(S)-1-Ethoxycarbonyl-butyl]-L-alanyl]-(1SR,3SR,5SR)-2-azabicyclo[3.3.0]-7-octen-3-carbonsäurehydrochlorid

a) 2-[N-[(S)-1-Ethoxycarbonyl-butyl]-L-alanyl]-(1SR,3SR,5SR)-2-azabicyclo[3.3.0]-7-octen-3-carbonsäure-tert.-butylester

2,9 g N-[(S)-1-Ethoxycarbonyl-butyl]-L-alanin (Tetrahedron Letters 23, 1677 (1982) und 1 Äquivalent (1SR,3SR,5SR)-2-azabicyclo[3.3.0]-7-octen-3-carbonsäure-tert.-butylester aus Beispiel 1 d werden in 60 ml trockenem Methylenchlorid gelöst. Unter Eiskühlung werden 5,5 ml Triethylamin zugetropft. Danach werden 6,7 ml 50%ige n-Propanphosphonsäureanhydridlösung in Methylenchlorid zugegeben. Es wird 1 Stunde bei 0 ° C und weitere 14 Std. bei Raumtemperatur gerührt. Es wird wie in Beispiel 6 b beschrieben aufgearbeitet.
Rückstand: 5,0 g Öl

$^1$H-NMR: 0,9 (t, 3H);
1,25 (t, 3H);
1,4 (s, 9H);
0,9 - 3,8 (m, 12H);
3,9 - 4,7 (m, 5H);
5,4 - 6,2 (m, 2H).

Der Rückstand besteht aus dem Diastereomerengemisch 2-[N-[(S)-1-Ethoxycarbonyl-butyl]-L-alanyl]-(1S,3S,5S)-2-azabicyclo[3.3.0]-7-octen-3-carbonsäure-tert.-butylester und 2-[N-[(S)-1-Ethoxycarbonyl-butyl]-L-alanyl]-(1R,3R,5R)-2-azabicyclo[3.3.0]-7-octen-3-carbonsäure-tert.-butylester, das über Kieselgel mit dem Laufmittelgemisch Cyclohexan/Essigester 1:1 aufgetrennt werden kann.

b) 2-[N-[(S)-1-Ethoxycarbonyl-butyl]-L-alanyl]-(1SR,3SR,5SR)-2-azabicyclo[3.3.0]-7-octen-3-carbonsäurehydrochlorid

1,0 g des Rückstands aus Beispiel 8 a wird wie in Beispiel 1 h beschrieben umgesetzt.
Ausbeute: 0,8 g (m/e: 352)

Beispiel 9

2-[N-[(S)-1-Carboxy-butyl]-L-alanyl]-(1SR,3SR,5SR)-2-azabicyclo[3.3.0]-7-octen-3-carbonsäure

0,5 g der Verbindung aus Beispiel 8 b werden analog der Vorschrift von Beispiel 3 umgesetzt.
Ausbeute: 0,3 g (m/e: 306, MG-$H_2O$)

Beispiel 10

2-[N-[(S)-1-Ethoxycarbonyl-3-cyclohexylpropyl]-L-alanyl]-(1R,3S,5R)-2-azabicyclo[3.3.0]-7-octen-3-carbonsäure

a) 2-[N-[(S)-1-Ethoxycarbonyl-3-cyclohexylpropyl]-L-alanyl]-(1R,3S,5R)-2-azabicyclo[3.3.0]-7-octen-3-carbonsäure-tert.butylester

1,0 g des tert. Butylesters aus Beispiel 2d werden wie in Beispiel 1c beschrieben mit 1,3 g N-(1-S-Ethoxycarbonyl-3-cyclohexylpropyl-S-alanin umgesetzt.

Ausbeute 2,0 g Diastereomerengemisch, das über Kieselgel mit Petrolether/Aceton 2:1 säulenchromatographisch getrennt wird.
Ausbeute der im Titel genannten Verbindung: 0,9 g; $[\alpha]_D^{20}$ : -98 ° (c = 6, Essigester).

Die isomere Verbindung 2-[N-[-(S)-1-Ethoxycarbonyl-3-cyclohexylpropyl]-L-alanyl]-(1S,3R,5S)-2-azabicyco [3.3.0]-7-octen-3-carbonsäure-tert.butylester zeigt $[\alpha]_D^{20}$ : + 75 ° (c = 5, Essigester).

b) 2-[N-[(S)-1-Ethoxycarbonyl-3-cyclohexylpropyl]-L-alanyl]-(1R,3S,5R)-2-azabicyclo[3.3.0]-7-octen-3-carbonsäure

0,5 g der Verbindung aus Beispiel 10 a mit der S,S,S, S,S-Konfiguration werden wie in Beispiel 1h) beschrieben umgesetzt.
Ausbeute: 0,2 g; $[\alpha]_D^{20}$ : -84,6 ° (c = 1, $CH_3OH$)

Die isomere Verbindung mit der S,R,S,S,S-Konfiguration, 2-[N-[(S)-1-Ethoxycarbonyl-3-cyclohexylpropyl]-L-alanyl]-(1S,3R,5S)-2-azabicyclo[3.3.0]-7-octen-3-carbonsäure, zeigt $[\alpha]_D^{20}$ : + 84 ° (c = 3, $CH_3OH$).

Beispiel 11

2-[N-[(S)-1-carboxy-3-cyclohexylpropyl]-L-alanyl]-(1R, 3S,5R)-2-azabicyclo[3.3.0]-7-octen-3-carbonsäure

0,1 g der Verbindung aus Beispiel 10 b (all-S-Konfiguration) werden wie in Beispiel 3 beschrieben, umgesetzt. Ausbeute: 0,6 g (m/e: 392).

**Patentansprüche**

**Patentansprüche für folgende Vertragsstaaten : BE, CH, DE, FR, GB, IT, LI, LU, NL, SE**

**1.** Verbindungen der Formel I,

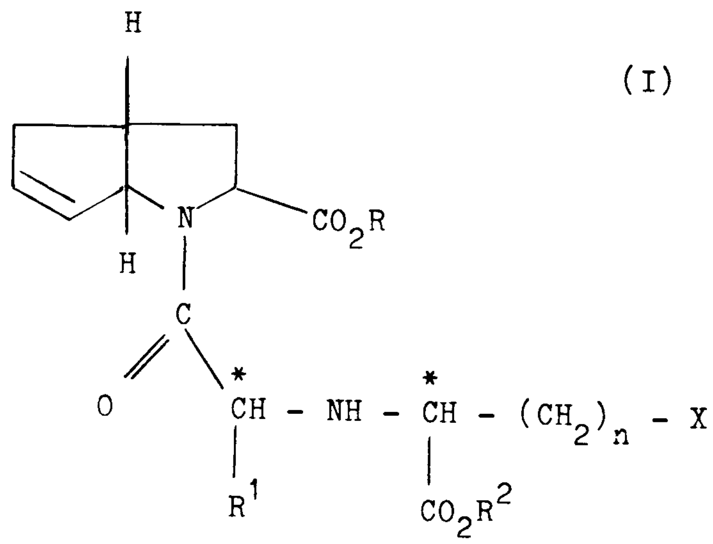

(I)

in der

n = 0, 1 oder 2,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$-Cycloalkyl-($C_1$ bis $C_4$)-alkyl, Phenyl, Naphtyl oder teilhydriertes Phenyl oder Naphtyl, das jeweils durch ($C_1$ bis $C_4$)-Alkyl, ($C_1$ oder $C_2$)-Alkoxy oder Halogen substituiert sein kann, Aryl-($C_1$ bis $C_4$)-alkyl oder Aroyl-$C_1$-alkyl, die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, ($C_1$ bis $C_6$)-Alkyl, ($C_2$ bis $C_6$)-Alkenyl oder Aryl-($C_1$ bis $C_4$)-alkyl,

X = ($C_1$ bis $C_6$)-Alkyl, ($C_2$ bis $C_6$)-Alkenyl, ($C_5$ bis $C_9$)-Cycloalkyl, Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino oder Methylendioxy mono-, di- oder trisubstituiert sein kann, oder 3-Indolyl bedeuten,

sowie deren physiologisch unbedenkliche Salze.

**2.** Verbindungen der Formel I gemäß Anspruch 1, dadurch gekennzeichnet, daß die Wasserstoffatome an den Brückenkopf-C-Atomen 1 und 5 zueinander cis-konfiguriert sind.

**3.** Verbindungen der Formel I gemäß den Ansprüchen 1 und 2, dadurch gekennzeichnet, daß das C-Atom in Position 3 des bicyclischen Systems sowie die mit einem Stern markierten C-Atome der Seitenkette jeweils S-Konfiguration aufweisen.

**4.** Verbindungen der Formel I gemäß Ansprüchen 1 bis 3, dadurch gekennzeichnet, daß

n = 2

R = Wasserstoff oder ($C_1$ bis $C_4$)-Alkyl,

$R^1$ = Wasserstoff, ($C_1$ bis $C_3$)-Alkyl, ($C_2$ oder $C_3$)-Alkenyl, Benzyl, Phenethyl, 4-Amino-butyl oder Benzoylmethyl

$R^2$ = Wasserstoff, ($C_1$ bis $C_4$)-Alkyl oder Benzyl,

X = Methyl, Cyclohexyl, Phenyl, das durch $C_1$- oder $C_2$-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkylamino, Nitro oder Methylendioxy mono- oder disubsituiert oder, im Falle von Methoxy, trisubstituiert sein kann, bedeuten.

**5.** Verbindungen der Formel I gemäß den Ansprüchen 1 bis 4, dadurch gekennzeichnet, daß R = Wasserstoff, $R^1$ = Methyl und X = Phenyl, Methyl oder Cyclohexyl bedeuten und/oder $R^2$ = Wasserstoff oder Ethyl bedeutet, und/oder X = Phenyl bedeutet.

**6.** Verbindung der Formel I gemäß Anspruch 2, dadurch gekennzeichnet, daß die Carboxygruppe am C-Atom in Position 3 des bicyclischen Systems exo- oder endoständig, vorzugsweise endoständig orientiert ist und

das vorstehend genannte C-Atom sowie die mit einem Stern markierten C-Atome der Seitenkette jeweils S-Konfiguration aufweisen,

n = 2,

R = Wasserstoff,

$R^1$ = Methyl,

$R^2$ = Ethyl und

X = Phenyl bedeuten.

**7.** Verfahren zur Herstellung der Verbindungen der Formel I gemäß den Ansprüchen 1 bis 6, dadurch gekennzeichnet,

a) daß man eine Verbindung der Formel II

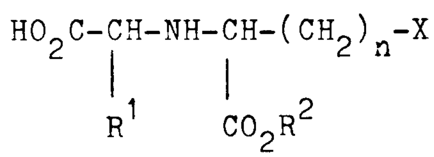

(II)

worin n, $R^1$, $R^2$ und X die zur Formel I genannten Bedeutungen hat, mit einer Verbindung der Formel III,

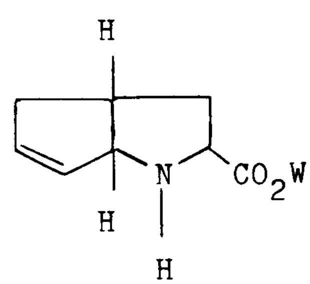

(III)

in welcher

W = Wasserstoff oder einen sauer abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I

$b_1$) eine Verbindung der Formel IV,

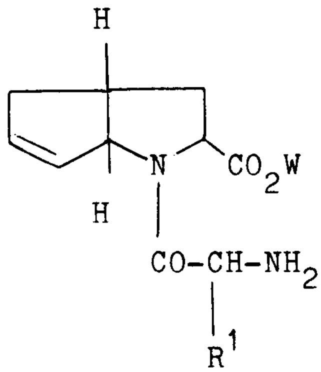

(IV)

in welcher $R^1$ die Bedeutung wie in Formel I und W die Bedeutung wie in Formel III besitzen, mit einer Verbindung der Formel XII,

$R^2O_2C\text{-}CH = CH\text{-}CO\text{-}X$ (XII)

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindung der unter $b_1$) genannten Formel IX mit einer Verbindung der allgemeinen Formel VIII, worin $R^2$ die Bedeutung wie in Formel I hat, und mit einer Verbindung der allgemeinen Formel VII,

$OHC\text{-}CO_2R^2$ (IX)

$X\text{-}CO\text{-}CH_3$ (VIII)

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I eine Verbindung der unter $b_1$) genannten Formel IV mit einer Verbindung der Formel V,

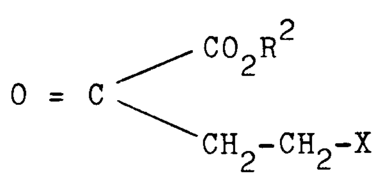

(V)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet und die nach a) bis c) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt und die erhaltenen Verbindungen ggf. in ihre physiologisch unbedenklichen Salze überführt.

**8.** Mittel, enthaltend eine Verbindung gemäß Ansprüchen 1 bis 6.

**9.** Verbindungen der Formel III,

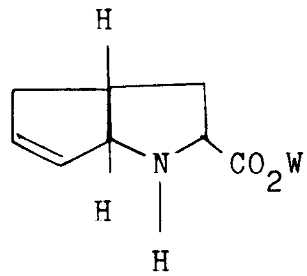

(III)

in der die H-Atome an den C-Atomen 1 und 5 zueinander cis-konfiguriert sind und die Gruppe $-CO_2W$ an C-Atom 3 exo- oder endo-ständig zum bicyclischen Ringsystem orientiert ist und worin

W = Wasserstoff oder einen sauer abspaltbaren Rest bedeutet.

**10.** Verfahren zur Herstellung von Verbindungen der Formel III gemäß Anspruch 10, dadurch gekennzeichnet, daß man
Verbindungen der Formel VI a oder VII a

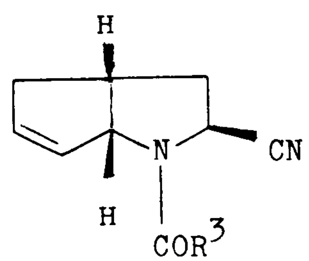

VI a

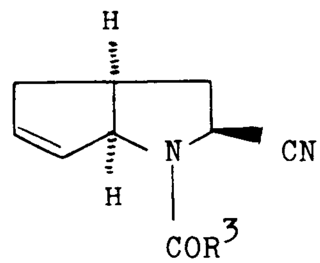

VII a

worin die H-Atome an den C-Atomen 1 und 5 zueinander cis-konfiguriert sind und die Nitrilgruppe an C-Atom 3 exo- oder endoständig zum bicyclischen Ringsystem orientiert ist und worin $R^3$ für $(C_1-C_6)$-Alkyl, $(C_5-C_9)$-Cycloalkyl, $(C_2-C_6)$-Alkenyl, $(C_1-C_6)$-Alkoxy, Aryl, Aryloxy, Aryl-$(C_1-C_4)$-alkoxy steht, gegebenenfalls als Racemate, zu Verbindungen der Formel III a bzw. III b sauer oder alkalisch

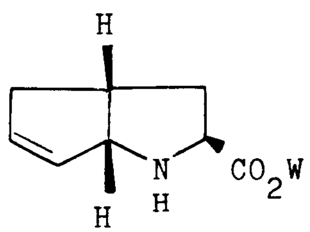

(III a)

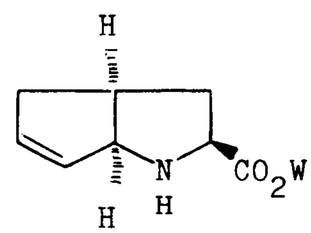

(III b)

hydrolysiert, wobei W Wasserstoff bedeutet und die Wasserstoffatome an den C-Atomen 1 und 5 zueinander cis-konfiguriert sind und die $CO_2W$-Gruppe an C-Atom 3 exo-ständig (III a) oder endoständig (III b) zum olefinischen Fünfring steht und die erhaltenen Racemate gegebenenfalls nach vorheriger Auftrennung in Enantiomere zu Verbindungen der Formel III a und III b, in welchen W für einen sauer abspaltbaren Rest steht, umsetzt.

**11.** Mittel, enthaltend eine Verbindung gemäß Ansprüchen 1 bis 6 in Kombination mit einem Diuretikum.

**Patentansprüche für folgenden Vertragsstaat : AT**

**1.** Verfahren zur Herstellung der Verbindungen der Formel I,

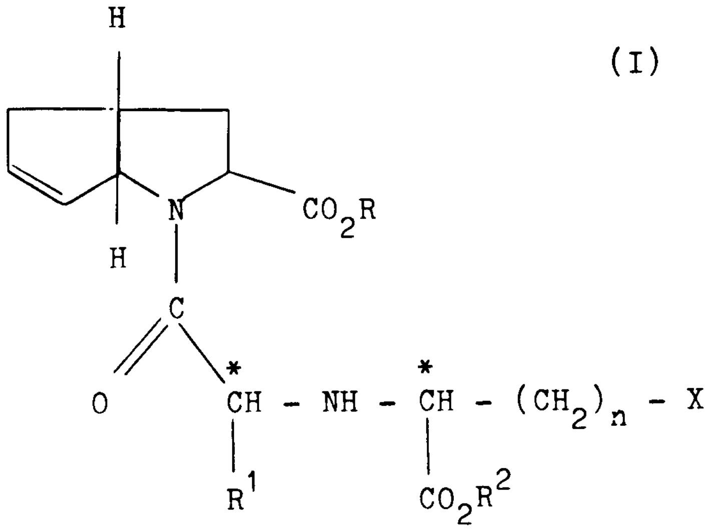

(I)

in der

n = 0, 1 oder 2,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$-Cycloalkyl-($C_1$ bis $C_4$)-alkyl, Phenyl, Naphtyl oder teilhydriertes Phenyl oder Naphtyl, das jeweils durch ($C_1$ bis $C_4$)-Alkyl, ($C_1$ oder $C_2$)-Alkoxy oder Halogen substituiert sein kann, Aryl-($C_1$ bis $C_4$)-alkyl oder Aroyl-$C_1$-alkyl, die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, ($C_1$ bis $C_6$)-Alkyl, ($C_2$ bis $C_6$)-Alkenyl oder Aryl-($C_1$ bis $C_4$)-alkyl,

X = ($C_1$ bis $C_6$)-Alkyl, ($C_2$ bis $C_6$)-Alkenyl, ($C_5$ bis $C_9$)-Cycloalkyl, Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino oder Methylendioxy mono-, di- oder trisubstituiert sein kann, oder 3-Indolyl bedeuten,

sowie deren physiologisch unbedenkliche Salze, dadurch gekennzeichnet,

a) daß man eine Verbindung der Formel II

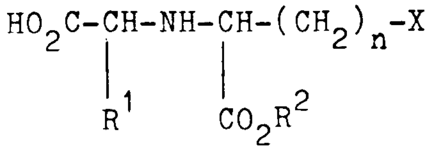

(II)

worin n, $R^1$, $R^2$ und X die zur Formel I genannten Bedeutungen hat, mit einer Verbindung der Formel III,

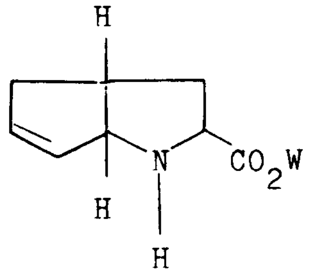

(III)

in welcher

W = Wasserstoff oder einen sauer abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I

$b_1$) eine Verbindung der Formel IV,

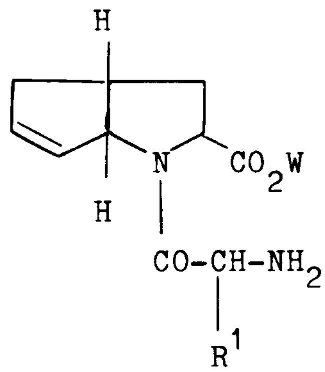

(IV)

in welcher $R^1$ die Bedeutung wie in Formel I und W die Bedeutung wie in Formel III besitzen, mit einer Verbindung der Formel XII,

$R^2O_2C\text{-}CH=CH\text{-}CO\text{-}X$ (XII)

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindung der unter $b_1$) genannten Formel IX mit einer Verbindung der allgemeinen Formel VIII, worin $R^2$ die Bedeutung wie in Formel I hat, und mit einer Verbindung der allgemeinen Formel VII,

$OHC\text{-}CO_2R^2$ (IX) $X\text{-}CO\text{-}CH_3$ (VIII)

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I eine Verbindung der unter $b_1$) genannten Formel IV mit einer Verbindung der Formel V,

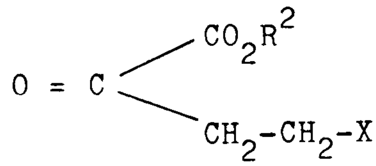

(V)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet und die nach a) bis c) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt und die erhaltenen Verbindungen ggf. in ihre physiologisch unbedenklichen Salze überführt.

**2.** Verfahren gemäß Anspruch 1, dadurch gekennzeichnet, daß man Verbindungen der Formel I herstellt, worin die Wasserstoffatome an den Brückenkopf-C-Atomen 1 und 5 zueinander cis-konfiguriert sind.

**3.** Verfahren gemäß den Ansprüchen 1 und 2, dadurch gekennzeichnet, daß man Verbindungen der Formel I herstellt, worin das C-Atom in Position 3 des bicyclischen Systems sowie die mit einem Stern markierten C-Atome der Seitenkette jeweils S-Konfiguration aufweisen.

**4.** Verfahren gemäß Ansprüchen 1 bis 3, dadurch gekennzeichnet, daß man Verbindungen der Formel I herstellt, worin

| | |
|---|---|
| n = | 2 |
| R = | Wasserstoff oder ($C_1$ bis $C_4$)-Alkyl, |
| $R^1$ = | Wasserstoff, ($C_1$ bis $C_3$)-Alkyl, ($C_2$ oder $C_3$)-Alkenyl, Benzyl, Phenethyl, 4-Amino-butyl oder Benzoylmethyl |
| $R^2$ = | Wasserstoff, ($C_1$ bis $C_4$)-Alkyl oder Benzyl, |
| X = | Methyl, Cyclohexyl, Phenyl, das durch $C_1$- oder $C_2$-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkylamino, Nitro oder Methylendioxy mono- oder disubsituiert oder, im Falle von Methoxy, trisubstituiert sein kann, bedeuten. |

**5.** Verfahren gemäß den Ansprüchen 1 bis 4, dadurch gekennzeichnet, daß man Verbindungen der Formel I herstellt, worin R = Wasserstoff, $R^1$ = Methyl und X = Phenyl, Methyl oder Cyclohexyl bedeuten, und/oder $R^2$ = Wasserstoff oder Ethyl bedeutet, und/oder X = Phenyl bedeutet.

**6.** Verfahren gemäß Anspruch 2, dadurch gekennzeichnet, daß man Verbindungen der Formel I herstellt, worin die Carboxygruppe am C-Atom in Position 3 des bicyclischen Systems exo- oder endoständig, vorzugsweise endoständig orientiert ist und

das vorstehend genannte C-Atom sowie die mit einem Stern markierten C-Atome der Seitenkette jeweils S-Konfiguration aufweisen,

| | |
|---|---|
| n = | 2, |
| R = | Wasserstoff, |
| $R^1$ = | Methyl, |
| $R^2$ = | Ethyl und |
| X = | Phenyl bedeuten. |

**7.** Verfahren zur Herstellung eines Mittels, enthaltend eine Verbindung hergestellt gemäß Ansprüchen 1 bis 6, dadurch gekennzeichnet, daß man diese in eine geeignete Darreichungsform bringt.

**8.** Verfahren zur Herstellung von Verbindungen der Formel III

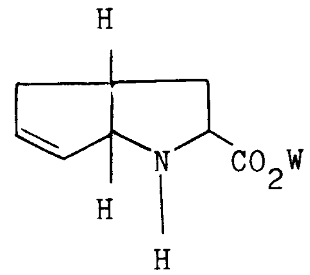

(III)

in der die H-Atome an den C-Atomen 1 und 5 zueinander cis-konfiguriert sind und die Gruppe $-CO_2W$ an C-Atom 3 exo- oder endo-ständig zum bicyclischen Ringsystem orientiert ist und worin

W = Wasserstoff oder einen sauer abspaltbaren Rest bedeutet, dadurch gekennzeichnet, daß man

Verbindungen der Formel VI a oder VII a

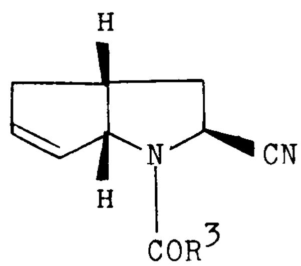

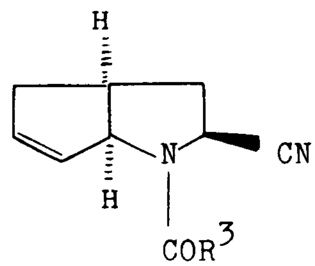

VI a VII a

worin die H-Atome an den C-Atomen 1 und 5 zueinander cis-konfiguriert sind und die Nitrilgruppe an C-Atom 3 exo- oder endoständig zum bicyclischen Ringsystem orientiert ist und worin $R^3$ für $(C_1\text{-}C_6)$-Alkyl, $(C_5\text{-}C_9)$-Cycloalkyl, $(C_2\text{-}C_6)$-Alkenyl, $(C_1\text{-}C_6)$-Alkoxy, Aryl, Aryloxy, Aryl-$(C_1\text{-}C_4)$-alkoxy steht, gegebenenfalls als Racemate, zu Verbindungen der Formel III a bzw. III b sauer oder alkalisch

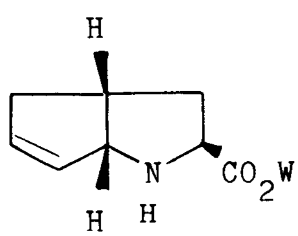

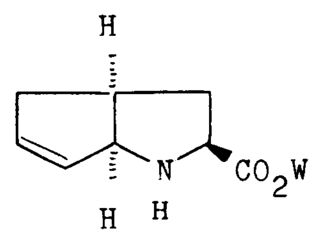

(III a) (III b)

hydrolysiert, wobei W Wasserstoff bedeutet und die Wasserstoffatome an den C-Atomen 1 und 5 zueinander cis-konfiguriert sind und die $CO_2W$-Gruppe an C-Atom 3 exo-ständig (III a) oder endo-ständig (III b) zum olefinischen Fünfring steht und die erhaltenen Racemate gegebenenfalls nach vorheriger Auftrennung in Enantiomere zu Verbindungen der Formel III a und III b, in welchen W für einen sauer abspaltbaren Rest steht, umsetzt.

**9.** Verfahren gemäß Anspruch 8 zur Herstellung eines Mittels, enthaltend eine Verbindung hergestellt gemäß Ansprüchen 1 bis 6 in Kombination mit einem Diuretikum.

**Claims**

**Claims for the following Contracting States : BE, CH, DE, FR, GB, IT, LI, LU, NL, SE**

**1.** A compound of the formula I

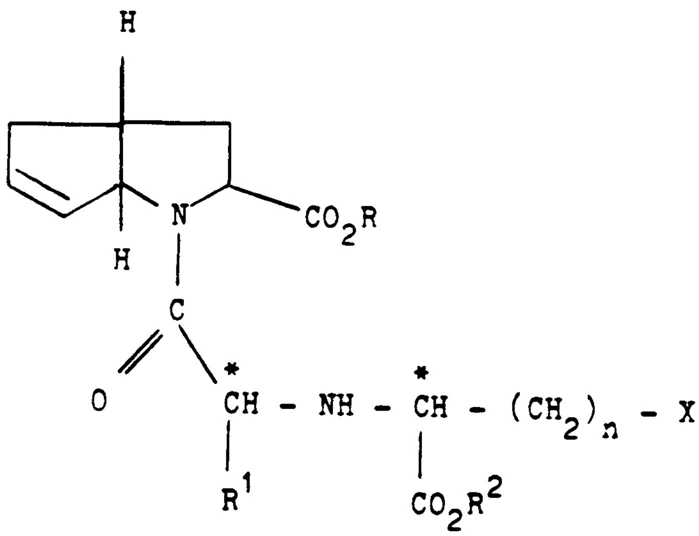

(I).

in which

- n denotes 0, 1 or 2,
- R denotes hydrogen, ($C_1$ to $C_6$)-alkyl or aralkyl having 7 to 9 carbon atoms,
- $R^1$ denotes hydrogen or ($C_1$ to $C_6$)-alkyl which can optionally be substituted by amino, ($C_1$ to $C_4$)-acylamino or benzoylamino, or ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, phenyl, naphthyl or partially hydrogenated phenyl or naphthyl, each of which can be substituted by ($C_1$ to $C_4$)-alkyl, ($C_1$ or $C_2$)-alkoxy or halogen, or aryl-($C_1$ to $C_4$)-alkyl or aroyl-$C_1$-alkyl, both of which can be substituted in the aryl radical as defined previously, a monocyclic or bicyclic heterocyclic radical having 5 to 7 or 8 to 10 ring atoms, 1 or 2 of these ring atoms being sulfur or oxygen atoms and/or 1 to 4 of these ring atoms being nitrogen atoms, or a side chain of a naturally occurring amino acid,
- $R^2$ denotes hydrogen, ($C_1$ to $C_6$)-alkyl, ($C_2$ to $C_6$)-alkenyl or aryl-($C_1$ to $C_4$)-alkyl,
- X denotes ($C_1$ to $C_6$)-alkyl, ($C_2$ to $C_6$)-alkenyl, ($C_5$ to $C_9$)-cycloalkyl, aryl which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino or methylenedioxy, or 3-indolyl,

and the physiologically acceptable salts thereof.

**2.** A compound of the formula I as claimed in claim 1, wherein the hydrogen atoms on the bridgehead carbon atoms 1 and 5 have the cis configuration with respect to one another.

**3.** A compound of the formula I as claimed in claims 1 and 2, wherein the carbon atom in position 3 of the bicyclic system, and the carbon atoms in the side chain which are labeled with an asterisk, each have the S configuration.

**4.** A compound of the formula I as claimed in claims 1 to 3, wherein

- n denotes 2,
- R denotes hydrogen or ($C_1$ to $C_4$)-alkyl,
- $R^1$ denotes hydrogen, ($C_1$ to $C_3$)-alkyl, ($C_2$ or $C_3$)-alkenyl, benzyl, phenethyl, 4-aminobutyl or benzoylmethyl,
- $R^2$ denotes hydrogen, ($C_1$ to $C_4$)-alkyl or benzyl and
- X denotes methyl, cyclohexyl, phenyl which can be monosubstituted or disubstituted or, in the case of methoxy, trisubstituted by $C_1$- or $C_2$-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro or

methylenedioxy.

**5.** A compound of the formula I as claimed in claims 1 to 4, wherein R denotes hydrogen, $R^1$ denotes methyl and X denotes phenyl, methyl or cyclohexyl and/or
$R^2$ denotes hydrogen or ethyl, and/or
X denotes phenyl.

**6.** A compound of the formula I as claimed in claim 2, wherein the carboxyl group on the carbon atom in position 3 of the bicyclic system has the exo or endo orientation, preferably the endo orientation, and the abovementioned carbon atom and the carbon atoms in the side chain which are labeled with an asterisk each have the S configuration,
n denotes 2,
R denotes hydrogen,
$R^1$ denotes methyl,
$R^2$ denotes ethyl and
X denotes phenyl.

**7.** A process for the preparation of the compounds of the formula I as claimed in claims 1 to 6, which comprises
a) reaction of a compound of the formula II

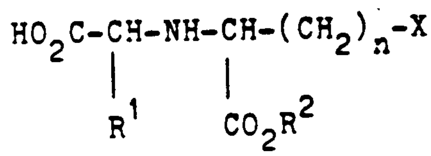

(II)

in which n, $R^1$, $R^2$ and X have the meanings mentioned for formula I, with a compound of the formula III

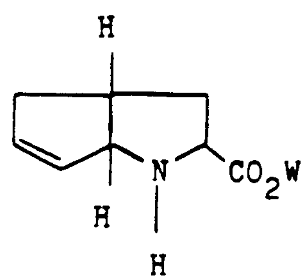

(III)

in which
W denotes hydrogen or a radical which can be eliminated with acid, and then, where appropriate, elimination of W and/or $R^2$ with formation of the free carboxyl groups, or
b) for the preparation of the compounds of the formula I
$b_1$) reaction of a compound of the formula IV

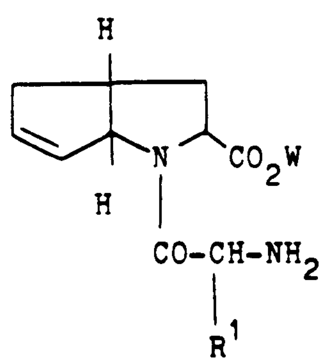

(IV)

in which $R^1$ has the meaning as in formula I and W has the meaning as in formula III, with a compound of the formula XII

$R^2O_2C\text{-}CH=CH\text{-}CO\text{-}X$ (XII)

in which $R^2$ and X have the meanings as in formula I, and then, where appropriate, elimination of W and/or $R^2$ with formation of the free carboxyl groups, or

$b_2$) reaction of a compound of the formula I mentioned under $b_1$) with a compound of the general formula, in which $R^2$ has the meaning as in formula I, and with a compound of the general formula VII,

$OHC\text{-}CO_2R^2$ (IX) $X\text{-}CO\text{-}CH_3$ (VIII)

in which X has the meaning as in formula I, and then, where appropriate, elimination of W and/or $R^2$ with formation of the free carboxyl groups, or

c) for the preparation of compounds of the formula I, reaction of a compound of the formula IV mentioned under $b_1$) with a compound of the formula V

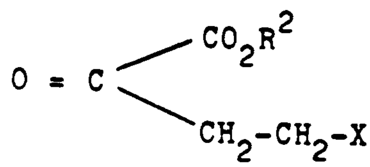

(V)

in which $R^2$ and X have the meanings as in formula I, reduction of the resulting Schiff's bases and then, where appropriate, elimination of W and/or $R^2$ with formation of the free carboxyl groups, and, where appropriate, conversion of the compounds of the formula I, obtained as in a) to c), in which R represents hydrogen, into esters of the formula I in which R denotes ($C_1$ to $C_6$)-alkyl or ($C_7$ to $C_9$)-aralkyl and conversion of the compounds obtained, where appropriate, into their physiologically acceptable salts.

**8.** An agent containing a compound as claimed in claims 1 to 6.

**9.** A compound of the formula III

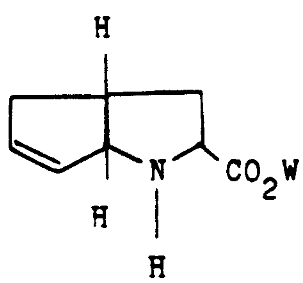

(III)

in which the hydrogen atoms on the carbon atoms 1 and 5 have the cis configuration with respect to one another, and the group $-CO_2W$ on carbon atom 3 is oriented exo or endo with respect to the bicyclic ring system, and in which W denotes hydrogen or a radical which can be eliminated with acid.

**10.** A process for the preparation of compounds of the formula III as claimed in claim 9, which comprises subjecting compounds of the formula VI a or VII a

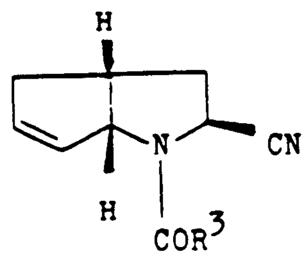

VIa

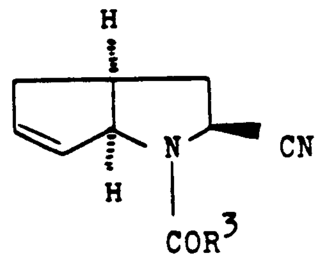

VIIa

in which the hydrogen atoms on the carbon atoms 1 and 5 have the cis configuration with respect to one another, and the nitrile group on carbon atom 3 is oriented exo or endo with respect to the bicyclic ring system, and in which $R^3$ represents $(C_1-C_6)$-alkyl,$(C_5-C_9)$-cycloalkyl, $(C_2-C_6)$-alkenyl, $(C_1-C_6)$-alkoxy, aryl, aryloxy or aryl-$(C_1-C_4)$-alkoxy, where appropriate as racemate, to acid or alkaline hydrolysis to give compounds of the formula III a or III b

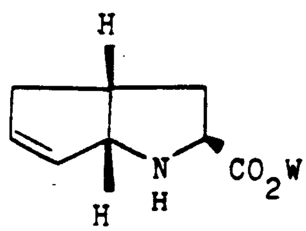

(IIIa)

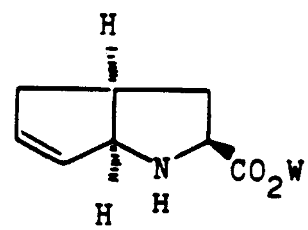

(IIIb)

W denoting hydrogen and the hydrogen atoms on carbon atoms 1 and 5 having the cis configuration with respect to one another, and the $CO_2W$ group on carbon atom 3 being exo (III a) or endo (III b) with respect to the olefinic five-membered ring, and reaction of the resulting racemates, where appropriate after previous separation into enantiomers, to give compounds of the formula III a and III b in which W represents a radical which can be eliminated with acid.

**11.** An agent containing a compound as claimed in claims 1 to 6 in combination with a diuretic.

**Claims for the following Contracting State : AT**

**1.** A process for the preparation of compounds of the formula I

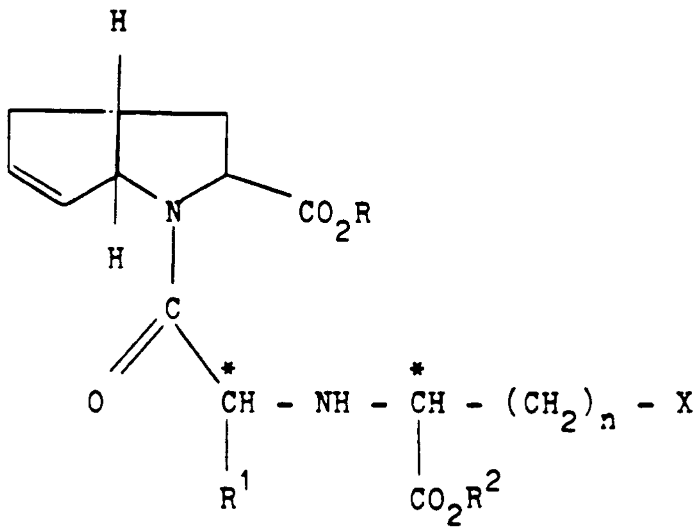

(I)

in which

- n denotes 0, 1 or 2,
- R denotes hydrogen, ($C_1$ to $C_6$)-alkyl or aralkyl having 7 to 9 carbon atoms,
- $R^1$ denotes hydrogen or ($C_1$ to $C_6$)-alkyl which can optionally be substituted by amino, ($C_1$ to $C_4$)-acylamino or benzoylamino, or ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, phenyl, naphthyl or partially hydrogenated phenyl or naphthyl, each of which can be substituted by ($C_1$ to $C_4$)-alkyl, ($C_1$ or $C_2$)-alkoxy or halogen, or aryl-($C_1$ to $C_4$)-alkyl or aroyl-$C_1$-alkyl, both of which can be substituted in the aryl radical as defined previously, a monocyclic or bicyclic heterocyclic radical having 5 to 7 or 8 to 10 ring atoms, 1 or 2 of these ring atoms being sulfur or oxygen atoms and/or 1 to 4 of these ring atoms being nitrogen atoms, or a side chain of a naturally occurring amino acid,
- $R^2$ denotes hydrogen, ($C_1$ to $C_6$)-alkyl, ($C_2$ to $C_6$)-alkenyl or aryl-($C_1$ to $C_4$)-alkyl,
- X denotes ($C_1$ to $C_6$)-alkyl, ($C_2$ to $C_6$)-alkenyl, ($C_5$ to $C_9$)-cycloalkyl, aryl which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino or methylenedioxy, or 3-indolyl,

and the physiologically acceptable salts thereof, which comprises

a) reaction of a compound of the formula II

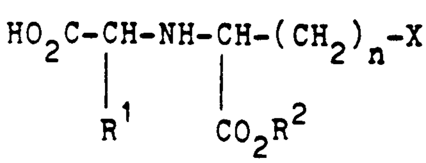

(II)

in which n, $R^1$, $R^2$ and X have the meanings mentioned for formula I, with a compound of the formula III

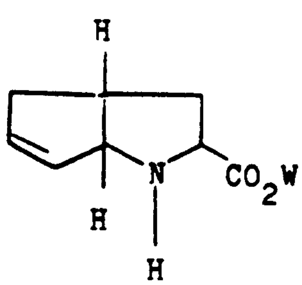

(III)

in which

W denotes hydrogen or a radical which can be eliminated with acid, and then, where appropriate, elimination of W and/or $R^2$ with formation of the free carboxyl groups, or

b) for the preparation of the compounds of the formula I

$b_1$) reaction of a compound of the formula IV

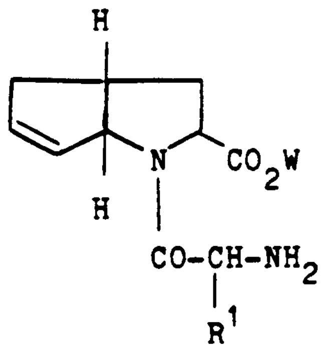

(IV)

in which $R^1$ has the meaning as in formula I and W has the meaning as in formula III, with a compound of the formula XII

$R^2O_2C\text{-}CH=CH\text{-}CO\text{-}X$ (XII)

in which $R^2$ and X have the meanings as in formula I, and then, where appropriate, elimination of W and/or $R^2$ with formation of the free carboxyl groups, or

$b_2$) reaction of a compound of the formula I mentioned under $b_1$) with a compound of the general formula, in which $R^2$ has the meaning as in formula I, and with a compound of the general formula VII,

| | |
|---|---|
| $OHC\text{-}CO_2R^2$ | $X\text{-}CO\text{-}CH_3$ |
| (IX) | (VIII) |

in which X has the meaning as in formula I, and then, where appropriate, elimination of W and/or $R^2$ with formation of the free carboxyl groups, or

c) for the preparation of compounds of the formula I, reaction of a compound of the formula IV mentioned under $b_1$) with a compound of the formula V

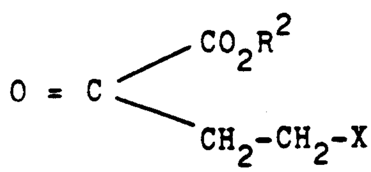

(V)

in which $R^2$ and X have the meanings as in formula I, reduction of the resulting Schiff's bases and then, where appropriate, elimination of W and/or $R^2$ with formation of the free carboxyl groups, and, where appropriate, conversion of the compounds of the formula I, obtained as in a) to c), in which R represents hydrogen, into esters of the formula I in which R denotes ($C_1$ to $C_6$)-alkyl or ($C_7$ to $C_9$)-aralkyl and conversion of the compounds obtained, where appropriate, into their physiologically acceptable salts.

2. The process as claimed in claim 1, wherein compounds of the formula I are prepared, in which the hydrogen atoms on the bridgehead carbon atoms 1 and 5 have the cis configuration with respect to one another.

3. The process as claimed in claims 1 and 2, wherein compounds of the formula I are prepared, in which

the carbon atom in position 3 of the bicyclic system, and the carbon atoms in the side chain which are labeled with an asterisk, each have the S configuration.

4. The process as claimed in claims 1 to 3, wherein compounds of the formula I are prepared, in which
   - n denotes 2,
   - R denotes hydrogen or ($C_1$ to $C_4$)-alkyl,
   - $R^1$ denotes hydrogen, ($C_1$ to $C_3$)-alkyl, ($C_2$ or $C_3$)-alkenyl, benzyl, phenethyl, 4-aminobutyl or benzoylmethyl,
   - $R^2$ denotes hydrogen, ($C_1$ to $C_4$)-alkyl or benzyl and
   - X denotes methyl, cyclohexyl, phenyl which can be monosubstituted or disubstituted or, in the case of methoxy, trisubstituted by $C_1$- or $C_2$-alkyl, ($C_1$ or $C_2$)-alkoxy, hydroxyl, fluorine, chlorine, bromine, amino, ($C_1$ to $C_4$)-alkylamino, di-($C_1$ to $C_4$)-alkylamino, nitro or methylenedioxy.

5. The process as claimed in claims 1 to 4, wherein compounds of the formula I are prepared, in which R denotes hydrogen, $R^1$ denotes methyl, and X denotes phenyl, methyl or cyclohexyl and/or $R^2$ denotes hydrogen or ethyl, and/or X denotes phenyl.

6. The process as claimed in claim 2, wherein compounds of the formula I are prepared, in which the carboxyl group on the carbon atom in position 3 of the bicyclic system has the exo or endo orientation, preferably the endo orientation, and the above-mentioned carbon atom and the carbon atoms in the side chain which are labeled with an asterisk each have the S configuration,
   - n denotes 2,
   - R denotes hydrogen,
   - $R^1$ denotes methyl,
   - $R^2$ denotes ethyl and
   - X denotes phenyl.

7. A process for the preparation of an agent containing a compound prepared as claimed in claims 1 to 6, which comprises the latter being converted into a suitable administration form.

8. A process for the preparation of compounds of the formula III

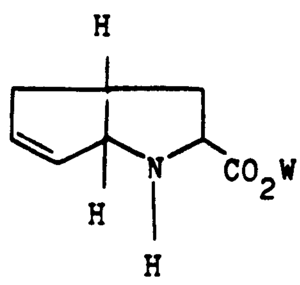

(III)

in which the hydrogen atoms on the carbon atoms 1 and 5 have the cis configuration in respect to one another, and the group $-CO_2W$ on carbon atom 3 is oriented exo or endo with respect to the bicyclic ring system, and in which W denotes hydrogen or a radical which can be eliminated with acid, which comprises subjecting compounds of the formula VI a or VII a

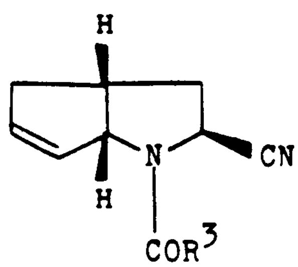

VIa

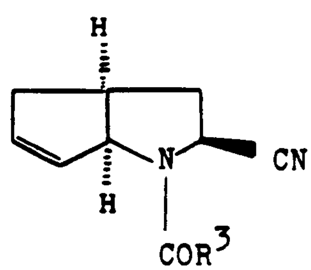

VIIa

in which the hydrogen atoms on the carbon atoms 1-and 5 have the cis configuration with respect to one another, and the nitrile group on carbon atom 3 is oriented exo or endo with respect to the bicyclic ring system, and in which $R^3$ represents $(C_1-C_6)$-alkyl,$(C_5-C_9)$-cycloalkyl, $(C_2-C_6)$-alkenyl, $(C_1-C_6)$-alkoxy, aryl, aryloxy or aryl-$(C_1-C_4)$-alkoxy, where appropriate as a racemate, to acid or alkaline hydrolysis to give compounds of the formula III a or III b

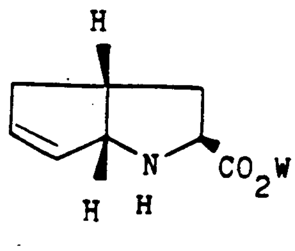

(IIIa)

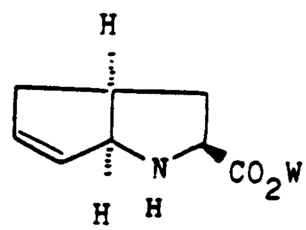

(IIIb)

W denoting hydrogen and the hydrogen atoms on carbon atoms 1 and 5 having the cis configuration with respect to one another, and the $CO_2W$ group on carbon atom 3 being exo (III a) or endo (III b) with respect to the olefinic five-membered ring, and reaction of the resulting racemates, where appropriate after previous separation into enantiomers, to give compounds of the formula III a and III b in which W represents a radical which can be eliminated with acid.

**9.** The process as claimed in claim 8 for the preparation of an agent containing a compound prepared as claimed in claims 1 to 6, in combination with a diuretic.

**Revendications**
**Revendications pour les Etats contractants suivants : BE, CH, DE, FR, GB, IT, LI, LU, NL, SE**

**1.** Composés de formule (I)

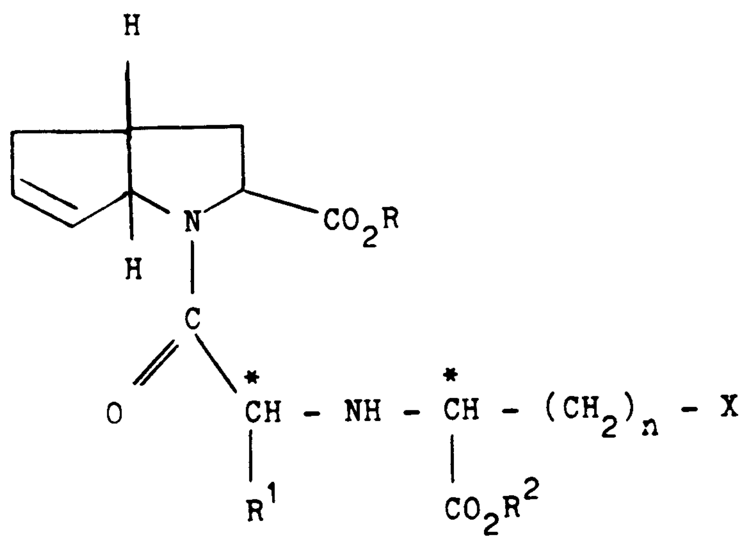

(I)

dans laquelle

n = 0, 1 ou 2;

R représente un atome d'hydrogène ou un groupe alkyle en $C_1$-$C_6$ ou aralkyle ayant de 7 à 9 atomes de carbone;

$R^1$ représente un atome d'hydrogène ou un radical alkyle en $C_1$-$C_6$ qui peut éventuellement être

substitué par un groupe amino, acyl($C_1$-$C_4$)-amino ou benzoylamino, un radical alcényle en $C_2$-$C_6$, cycloalkyle en $C_5$-$C_9$, cycloalcényle en $C_5$-$C_9$, cycloalkyl($C_5$-$C_7$)-alkyle($C_1$-$C_4$), phényle, naphtyle ou phényle ou napthyle partiellement hydrogénés, qui peut dans chaque cas être substitué par un groupe alkyle en $C_1$-$C_4$, alcoxy en $C_1$ ou $C_2$ ou par un halogène, un radical aryl-alkyle($C_1$-$C_4$) ou aroyl-alkyle($C_1$), qui peuvent l'un et l'autre être substitués sur le fragment aryle comme défini précédemment, un radical hétérocyclique mono- ou bicyclique ayant de 5 à 7, ou, respectivement, de 8 à 10 atomes formant le cycle, dont 1 ou 2 atomes formant le cycle représentent des atomes de soufre ou d'oxygène et/ou dont 1 à 4 atomes formant le cycle représentent des atomes d'azote, ou une chaîne latérale d'un aminoacide existant dans la nature;

$R^2$ représente un atome d'hydrogène ou un radical alkyle en $C_1$-$C_6$, alcényle en $C_2$-$C_6$ ou aryl-alkyle($C_1$-$C_4$);

X représente un radical alkyle en $C_1$-$C_6$, alcényle en $C_2$-$C_6$, cycloalkyle en $C_5$-$C_9$, aryle qui peut être mono-, di- ou trisubstitué par un ou des atomes d'halogène ou groupes alkyle en $C_1$-$C_4$, alcoxy en $C_1$-$C_4$, hydroxy, nitro, amino, alkyl($C_1$-$C_4$)-amino, dialkyl($C_1$-$C_4$)-amino ou méthylènedioxy, ou le radical 3-indolyle;

ainsi que leurs sels physiologiquement acceptables.

**2.** Composés de formule (I) selon la revendication 1, caractérisés en ce que les atomes d'hydrogène sur les atomes de carbone 1 et 5 de tête de pont sont en configuration cis l'un par rapport à l'autre.

**3.** Composés de formule (I) selon les revendications 1 et 2, caractérisés en ce que l'atome de carbone en position 3 du système bicyclique ainsi que les atomes de carbone de la chaîne latérale marqués par un astérisque présentent chacun la configuration S.

**4.** Composés de formule (I) selon les revendications 1 à 3, caractérisés en ce que

n = 2;

R représente un atome d'hydrogène ou un groupe alkyle ayant de 1 à 4 atomes de carbone;

$R^1$ représente un atome d'hydrogène ou un groupe alkyle en $C_1$-$C_3$, alcényle en $C_2$ ou $C_3$, benzyle, phénéthyle, 4-aminobutyle ou benzoylméthyle;

$R^2$ représente un atome d'hydrogène ou un groupe alkyle en $C_1$-$C_4$ ou benzyle;

X représente un radical méthyle, cyclohexyle, phényle qui peut être mono- ou bisubstitué par un ou des atomes de fluor, de chlore ou de brome ou groupes alkyle en $C_1$ ou $C_2$, alcoxy en $C_1$ ou $C_2$, hydroxy, amino, alkyl($C_1$-$C_4$)-amino, dialkyl($C_1$-$C_4$)-amino, nitro ou méthylènedioxy, ou trisubstitué dans le cas du groupe méthoxy.

**5.** Composés de formule (I) selon les revendications 1 à 4, caractérisés en ce que R représente un atome d'hydrogène, $R^1$ représente le groupe méthyle et X représente le groupe phényle, méthyle ou cyclohexyle, et/ou

$R^2$ représente un atome d'hydrogène ou le groupe éthyle, et/ou

X représente le groupe phényle.

**6.** Composé de formule (I) selon les revendications 2, caractérisé en ce que le groupe carboxy sur l'atome de carbone en position 3 du système bicyclique est orienté en position exo ou endo, de préférence endo, et l'atome de carbone mentionné précédemment ainsi que les atomes de carbone de la chaîne latérale marqués par un astérisque présentent chacun la configuration S,

n = 2,

R représente un atome d'hydrogène,

$R^1$ représente le groupe méthyle,

$R^2$ représente le groupe éthyle, et

X représente le groupe phényle.

**7.** Procédé de préparation des composés de formule (I) selon les revendications 1 à 6, caractérisé en ce que

a) on fait réagir un composé de formule (II)

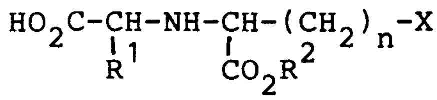

(II)

dans laquelle n, $R^1$, $R^2$ et X ont les significations données à propos de la formule (I), avec un composé de formule (III)

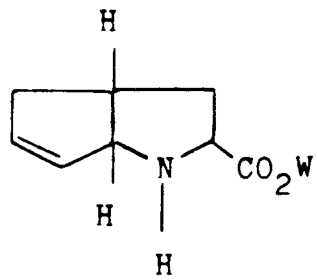

(III)

dans laquelle
W représente un atome d'hydrogène ou un radical séparable dans des conditions acides, et ensuite on élimine éventuellement W et/ou $R^2$ avec formation des groupes carboxy libres, ou
b) pour la préparation des composés de formule (I),
$b_1$) on fait réagir un composé de formule (IV)

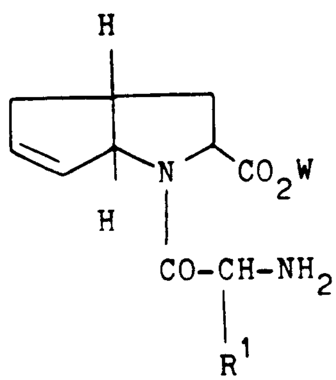

(IV)

dans laquelle $R^1$ a la même signification que dans la formule (I) et W a la même signification que dans la formule (III), avec un composé de formule (XII)

$R^2O_2C\text{-}CH=CH\text{-}CO\text{-}X$ (XII)

dans laquelle $R^2$ et X ont les mêmes significations que dans la formule (I), et ensuite éventuellement on élimine W et/ou $R^2$ avec formation des groupes carboxy libres, ou
$b_2$) on fait réagir un composé de formule (IV), mentionnée en $b_1$) avec un composé de formule générale (IX), dans laquelle $R^2$ a la même signification que dans la formule (I), et avec un composé de formule générale (VIII)

$OHC\text{-}CO_2R^2$ (IX)    $X\text{-}CO\text{-}CH_3$ (VIII)

dans laquelle X a la même signification que dans la formule (I), et ensuite éventuellement on élimine W et/ou $R^2$ avec formation des groupes carboxy libres, ou
c) pour la préparation de composés de formule (I), on fait réagir un composé de formule (IV),

mentionnée en $b_1$) avec un composé de formule (V)

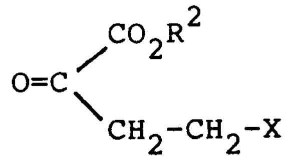

(V)

dans laquelle $R^2$ et X ont les mêmes significations que dans la formule (I), on réduit les bases de Schiff obtenues et ensuite éventuellement on élimine W et/ou $R^2$ avec formation des groupes carboxy libres, et on convertit éventuellement les composés de formule (I), dans lesquels R représente un atome d'hydrogène, obtenus selon a) à c), en esters de formule (I) dans lesquels R représente un groupe alkyle en $C_1$-$C_6$ ou aralkyle en $C_7$-$C_9$, et éventuellement on convertit les composés obtenus en leurs sels physiologiquement acceptables.

**8.** Médicaments contenant un composé selon les revendications 1 à 8.

**9.** Composés de formule (III)

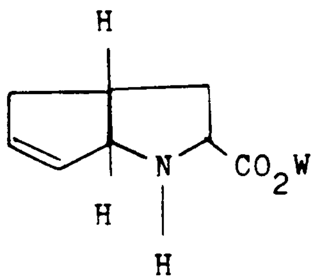

(III)

dans laquelle les atomes d'hydrogène sur les atomes de carbone 1 et 5 sont en configuration cis l'un par rapport à l'autre et le groupe $-CO_2W$ sur l'atome de carbone 3 est orienté en position exo ou endo par rapport au système bicyclique, et dans laquelle

W représente un atome d'hydrogène ou un radical séparable dans des conditions acides.

**10.** Procédé pour la préparation de composés de formule (III) selon la revendication 9, caractérisé en ce que l'on soumet à une hydrolyse acide ou alcaline des composés de formule (VIa) ou (VIIa)

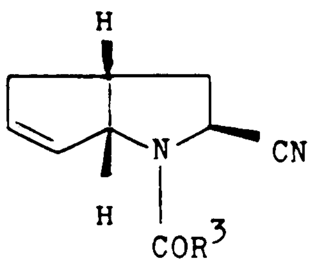

(VIa)

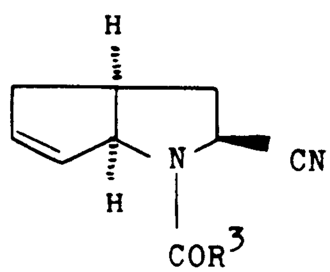

(VIIa)

dans lesquelles les atomes d'hydrogène sur les atomes de carbone 1 et 5 sont en configuration cis l'un par rapport à l'autre et le groupe nitrile sur l'atome de carbone 3 est orienté en position exo ou endo par rapport au système bicyclique, et dans lesquelles $R^3$ représente un groupe alkyle en $C_1$-$C_6$, cycloalkyle en $C_5$-$C_9$, alcényle en $C_2$-$C_6$, alcoxy en $C_1$-$C_6$, aryle, aryloxy, aryl-alcoxy($C_1$-$C_4$), éventuellement sous forme de racémiques, pour aboutir aux composés de formule (IIIa) ou (IIIb)

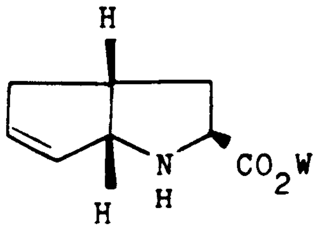

(IIIa)

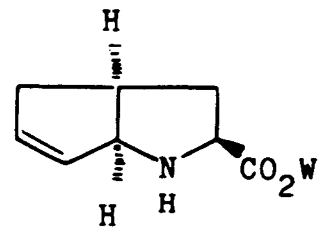

(IIIb)

W représentant un atome d'hydrogène et les atomes d'hydrogène sur les atomes de carbone 1 et 5 étant en configuration cis l'un par rapport à l'autre, et le groupe $CO_2W$ sur l'atome de carbone 3 étant en position exo (IIIa) ou endo (IIIb) par rapport au cycle pentagonal oléfinique, et on convertit les racémiques obtenus, éventuellement après dédoublement préliminaire en énantiomères, en composés de formules (IIIa) et (IIIb), dans lesquelles W représente un radical séparable dans des conditions acides.

**11.** Médicament contenant un composé selon les revendications 1 à 6, en association avec un diurétique.

**Revendications pour l'Etat contractant suivant : AT**

**1.** Procédé pour la préparation des composés de formule (I)

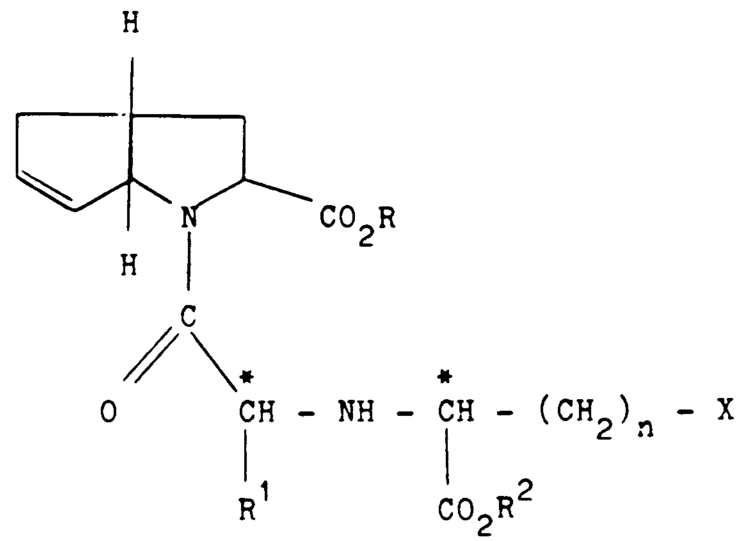

(I)

dans laquelle

n = 0, 1 ou 2;

R représente un atome d'hydrogène ou un groupe alkyle en $C_1$-$C_6$ ou aralkyle ayant de 7 à 9 atomes de carbone;

$R^1$ représente un atome d'hydrogène ou un radical alkyle en $C_1$-$C_6$ qui peut éventuellement être substitué par un groupe amino, acyl($C_1$-$C_4$)-amino ou benzoylamino, un radical alcényle en $C_2$-$C_6$, cycloalkyle en $C_5$-$C_9$, cycloalcényle en $C_5$-$C_9$, cycloalkyl($C_5$-$C_7$)-alkyle($C_1$-$C_4$), phényle, naphtyle ou phényle ou napthyle partiellement hydrogénés, qui peut dans chaque cas être substitué par un groupe alkyle en $C_1$-$C_4$, alcoxy en $C_1$ ou $C_2$ ou par un halogène, un radical aryl-alkyle($C_1$-$C_4$) ou aroyl-alkyle($C_1$), qui peuvent l'un et l'autre être substitués sur le fragment aryle comme défini précédemment, un radical hétérocyclique mono- ou bicyclique ayant de 5 à 7, ou, respectivement, de 8 à 10 atomes formant le cycle, dont 1 ou 2 atomes formant le cycle représentent des atomes de soufre ou d'oxygène et/ou dont 1 à 4 atomes formant le cycle représentent des atomes d'azote, ou une chaîne latérale d'un aminoacide existant dans la nature;

$R^2$ représente un atome d'hydrogène ou un radical alkyle en $C_1$-$C_6$, alcényle en $C_2$-$C_6$ ou aryl-

alkyle($C_1$-$C_4$);

X représente un radical alkyle en $C_1$-$C_6$, alcényle en $C_2$-$C_6$, cycloalkyle en $C_5$-$C_9$, aryle qui peut être mono-, di- ou trisubstitué par un ou des atomes d'halogène ou groupes alkyle en $C_1$-$C_4$, alcoxy en $C_1$-$C_4$, hydroxy, nitro, amino, alkyl($C_1$-$C_4$)-amino, dialkyl($C_1$-$C_4$)-amino ou méthylènedioxy, ou le radical 3-indolyle;

ainsi que de leurs sels physiologiquement acceptables, caractérisé en ce que

a) on fait réagir un composé de formule (II)

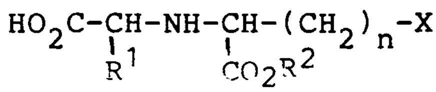

(II)

dans laquelle n, $R^1$, $R^2$ et X ont les significations données à propos de la formule (I), avec un composé de formule (III)

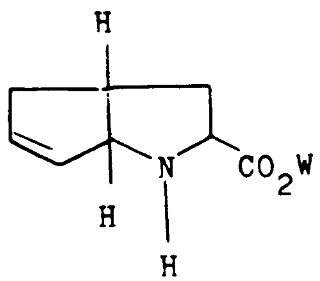

(III)

dans laquelle

W représente un atome d'hydrogène ou un radical séparable en milieu acide, et ensuite on élimine éventuellement W et/ou $R^2$ avec formation des groupes carboxy libres, ou

b) pour la préparation des composés de formule (I),

$b_1$) on fait réagir un composé de formule (IV)

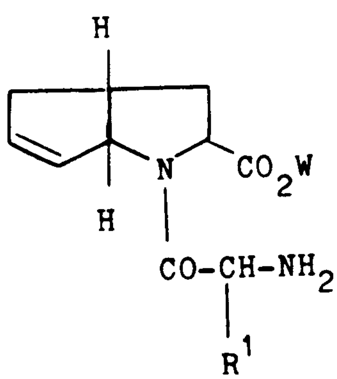

(IV)

dans laquelle $R^1$ a la même signification que dans la formule (I) et W a la même signification que dans la formule (III), avec un composé de formule (XII)

$R^2O_2C$-CH = CH-CO-X (XII)

dans laquelle $R^2$ et X ont les mêmes significations que dans la formule (I), et ensuite éventuellement on élimine W et/ou $R^2$ avec formation des groupes carboxy libres, ou

$b_2$) on fait réagir un composé de formule (IV), mentionnée en $b_1$) avec un composé de formule générale (IX), dans laquelle $R^2$ a la même signification que dans la formule (I), et avec un composé de formule générale (VIII)

$OHC-CO_2R^2$ (IX) $X-CO-CH_3$ (VIII)

dans laquelle X a la même signification que dans la formule (I), et ensuite éventuellement on élimine W et/ou $R^2$ avec formation des groupes carboxy libres, ou

c) pour la préparation de composés de formule (I), on fait réagir un composé de formule (IV), mentionnée en $b_1$) avec un composé de formule (V)

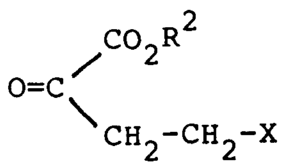

(V)

dans laquelle $R^2$ et X ont les mêmes significations que dans la formule (I), on réduit les bases de Schiff obtenues et ensuite éventuellement on élimine W et/ou $R^2$ avec formation des groupes carboxy libres, et on convertit éventuellement les composés de formule (I), dans lesquels R représente un atome d'hydrogène, obtenus selon a) à c), en esters de formule (I), dans lesquels R représente un groupe alkyle en $C_1$-$C_6$ ou aralkyle en $C_7$-$C_9$, et éventuellement on convertit les composés obtenus en leurs sels physiologiquement acceptables.

**2.** Procédé selon la revendication 1, caractérisé en ce que l'on prépare des composés de formule (I) dans lesquels les atomes d'hydrogène sur les atomes de carbone 1 et 5 de tête de pont sont en configuration cis l'un par rapport à l'autre.

**3.** Procédé selon les revendications 1 et 2, caractérisé en ce que l'on prépare des composés de formule (I) dans lesquels l'atome de carbone en position 3 du système bicyclique ainsi que les atomes de carbone de la chaîne latérale marqués par un astérisque présentent chacun la configuration S.

**4.** Procédé selon les revendications 1 à 3, caractérisé en ce que l'on prépare des composés de formule (I) dans lesquels

n = 2;
R représente un atome d'hydrogène ou un groupe alkyle ayant de 1 à 4 atomes de carbone;
$R^1$ représente un atome d'hydrogène ou un groupe alkyle en $C_1$-$C_3$, alcényle en $C_2$ ou $C_3$, benzyle, phénéthyle, 4-aminobutyle ou benzoylméthyle;
$R^2$ représente un atome d'hydrogène ou un groupe alkyle en $C_1$-$C_4$ ou benzyle;
X représente un radical méthyle, cyclohexyle, phényle qui peut être mono- ou bisubstitué par un ou des atomes de fluor, de chlore ou de brome ou groupes alkyle en $C_1$ ou $C_2$, alcoxy en $C_1$ ou $C_2$, hydroxy, amino, alkyl($C_1$-$C_4$)amino, dialkyl($C_1$-$C_4$)-amino, nitro ou méthylènedioxy, ou trisubstitué dans le cas du groupe méthoxy.

**5.** Procédé selon les revendications 1 à 4, caractérisé en ce que l'on prépare des composés de formule (I) dans lesquels R représente un atome d'hydrogène, $R^1$ représente le groupe méthyle et X représente le groupe phényle, méthyle ou cyclohexyle, et/ou
$R^2$ représente un atome d'hydrogène ou le groupe éthyle, et/ou
X représente le groupe phényle.

**6.** Procédé selon la revendication 2, caractérisé en ce que l'on prépare des composés de formule (I) dans lesquels le groupe carboxy sur l'atome de carbone en position 3 du système bicyclique est orienté en position exo ou endo, de préférence endo, et l'atome de carbone mentionné précédemment ainsi que les atomes de carbone de la chaîne latérale marqués par un astérisque présentent chacun la configuration S,

n = 2,
R représente un atome d'hydrogène,
$R^1$ représente le groupe méthyle,

$R^2$ représente le groupe éthyle, et
X représente le groupe phényle.

**7.** Procédé pour la fabrication d'un médicament contenant un composé préparé selon les revendications 1 à 6, caractérisé en ce que l'on met celui-ci sous une forme pharmaceutique appropriée.

**8.** Procédé pour la préparation de composés de formule (III)

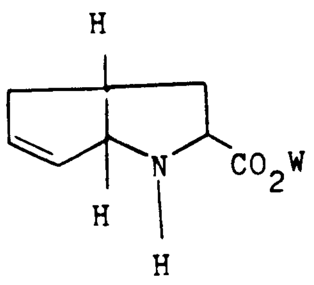

(III)

dans laquelle les atomes d'hydrogène sur les atomes de carbone 1 et 5 sont en configuration cis l'un par rapport à l'autre et le groupe $-CO_2W$ sur l'atome de carbone 3 est orienté en position exo ou endo par rapport au système bicyclique, et dans lesquels

W représente un atome d'hydrogène ou un radical séparable en milieu acide, caractérisé en ce que l'on soumet à une hydrolyse acide ou alcaline des composés de formule (VIa) ou (VIIa)

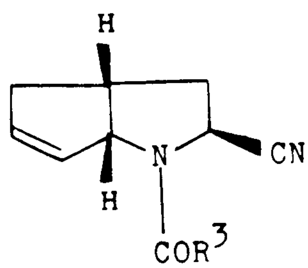

(VIa)

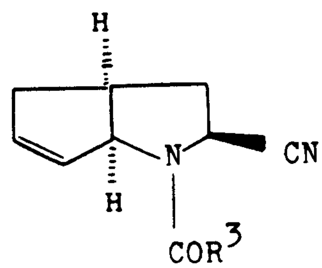

(VIIa)

dans lesquelles les atomes d'hydrogène sur les atomes de carbone 1 et 5 sont en configuration cis l'un par rapport à l'autre et le groupe nitrile sur l'atome de carbone 3 est orienté en position exo ou endo par rapport au système bicyclique, et dans lesquelles $R^3$ représente un groupe alkyle en $C_1$-$C_6$, cycloalkyle en $C_5$-$C_9$, alcényle en $C_2$-$C_6$, alcoxy en $C_1$-$C_6$, aryle, aryloxy, aryl-alcoxy($C_1$-$C_4$), éventuellement sous forme de racémiques, pour aboutir aux composés de formule (IIIa) ou (IIIb)

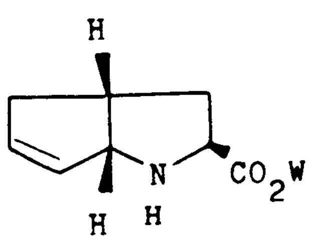

(IIIa)

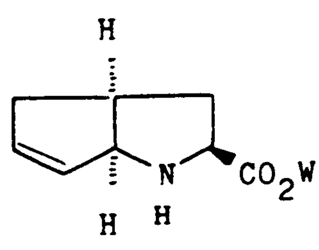

(IIIb)

W représentant un atome d'hydrogène et les atomes d'hydrogène sur les atomes de carbone 1 et 5 étant en configuration cis l'un par rapport à l'autre, et le groupe $CO_2W$ sur l'atome de carbone 3 étant en position exo (IIIa) ou endo (IIIb) par rapport au cycle pentagonal oléfinique, et on convertit les racémiques obtenus, éventuellement après dédoublement préliminaire en énantiomères, en composés de formules (IIIa) et (IIIb), dans lesquelles W représente un radical séparable en milieu acide.

9. Procédé selon la revendication 7, pour la fabrication d'un médicament contenant un composé préparé selon les revendications 1 à 6, en association avec un diurétique.